# EUROPÄISCHE PATENTANMELDUNG

(11) **EP 1 366 757 A1**
(43) Veröffentlichungstag der Anmeldung: **03.12.2003**
(21) Anmeldenummer: 03405346.2
(22) Anmeldetag: 20.05.2003
(51) Int. Cl.: A61K 7/48

(54) **Verwendung von Hydroxydiphenyletherverbindungen zur Behandlung der Haut**

(30) Priorität: 28.05.2002 EP 02405425; 19.02.2003 CH 252032003
(71) Anmelder: Ciba Specialty Chemicals Holding Inc., 4057 Basel (CH)
(72) Erfinder: Baschong, Werner, 4056 Basel (CH)

(57) **Zusammenfassung**

Beschrieben wird die Verwendung von
(a) Hydroxydiphenyletherverbindungen der Formel 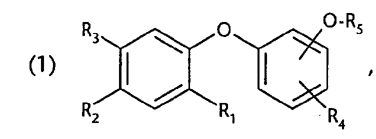 worin
R₁, R₂ und R₃ unabhängig voneinander Wasserstoff; Hydroxy; C₁-C₂₀-Alkyl; hydroxy-substituiertes C₁C₂₀-Alkyl; C₅-C₇-Cycloalkyl; C₁-C₂₀-Alkoxy; C₁-C₆-Alkylcarbonyl; Phenyl; oder Phenyl-C₁-C₃-Alkyl;
R₄ Wasserstoff, C₁-C₂₀Alkyl; hydroxy-substituiertes C₁-C₂₀-Alkyl; C₅-C₇-Cycloalkyl; Hydroxy; Formyl; Acetonyl; Allyl; Carboxy; Carboxy-C₁-C₃-Alkyl; Carboxyallyl; C₂-C₂₀-Alkenyl; C₁-C₆-Alkylcarbonyl; C₁-C₃-Alkylcarbonyl-C₁-C₃-Alkyl; Phenyl; oder Phenyl-C₁-C₃-Alkyl; und
R₅ Wasserstoff; C₁-C₂₀-Alkoxy; oder C₁-C₆-Alkylcarbonyl; bedeuten
als Melanogenese-Inhibitor und zum Aufhellen der Haut.

## Beschreibung

Die vorliegende Erfindung betrifft die Verwendung von Hydroxydiphenyletherverbindungen zur Behandlung, insbesondere zum Aufhellen der Haut und als Melanogenese-Inhibitor.

Es besteht ein starkes Interesse an Präparaten, die die Hautfarbe nach Sonneneinstrahlung nicht verändern und darüber hinaus in der Lage sind, durch Reduzierung der Produktion des Hautfarbstoffs Melanin bzw. durch dessen Aufhellung der Haut ein helleres Aussehen zu verleihen. Insbesondere in asiatischen Ländern ist ein heller Haut-Teint, d.h. eine Hautaufhellung oder Schutz vor Hautbräunung erwünscht.

Die Aufgabe der vorliegenden Erfindung besteht also darin, Zusammensetzungen zu finden, die eine Bräunung der Haut verhindern und gleichzeitig die Haut aufhellen können.

Überraschenderweise wurde nun gefunden, dass bestimmte Hydroxydiphenyletherverbindungen diese Aufgabe erfüllen können.

Gegenstand der vorliegenden Erfindung ist daher die Verwendung von
(a) Hydroxydiphenyletherverbindungen der Formel 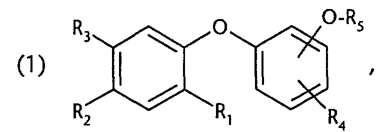
worin
- R₁, R₂ und R₃: unabhängig voneinander Wasserstoff; Hydroxy; C₁-C₂₀-Alkyl; hydroxy-substituiertes C₁C,₂₀-Alkyl; C₅-C₇-Cycloalkyl; C₁-C₂₀-Alkoxy; C₁-C₆-Alkylcarbonyl; Phenyl; oder Phenyl-C₁-C₃-Alkyl;
- R₄: Wasserstoff, C₁-C₂₀Alkyl; hydroxy-substituiertes C₁-C₂₀-Alkyl; C₅-C₇-Cycloalkyl; Hydroxy; Formyl; Acetonyl; Allyl; Carboxy; Carboxy-C₁-C₃-Alkyl; Carboxyallyl; C₂-C₂₀-Alkenyl; C₁-C₆-Alkylcarbonyl; C₁-C₃-Alkylcarbonyl-C₁-C₃-Alkyl; Phenyl; oder Phenyl-C₁-C₃-Alkyl; und
- R₅: Wasserstoff; C₁-C₂₀-Alkoxy; oder C₁-C₆-Alkylcarbonyl; bedeuten
als Melanogenese-Inhibitor und zum Aufhellen der Haut.

C₁-C₂₀-Alkyl bedeutet eine geradkettige oder verzweigte Alkylgruppe, Methyl, Ethyl, n-Propyl, Isopropyl, n-Butyl, sek.Butyl, tert.Butyl, Amyl, Isoamyl oder tert.Amyl, Hexyl, Heptyl, Octyl, Isooctyl, Nonyl, Decyl, Undecyl, Dodecyl, Tetradecyl, Pentadecyl, Hexadecyl, Heptadecyl oder Octadecyl.

C₁-C₂₀-Alkoxy bedeutet eine geradkettige oder verzweigte Alkoxygruppe, wie z.B. Methoxy, Ethoxy, n-Propoxy, lsopropoxy, n-Butoxy, sek.Butoxy, tert.Butoxy, Amyloxy, Isoamyloxy oder tert.Amyloxy, Hexyloxy, Heptyloxy, Octyloxy, Isooctyloxy, Nonyloxy, Decyloxy, Undecyloxy, Dodecyloxy, Tetradecyloxy, Pentadecyloxy, Hexadecyloxy, Heptadecyloxy oder Octadecyloxy.

C₂-C₁₈-Alkenyl bedeutet z.B. Allyl, Methallyl, Isopropenyl, 2-Butenyl, 3-Butenyl, Isobutenyl, n-Penta-2,4-dienyl, 3-Methyl-but-2-enyl, n-Oct-2-enyl, n-Dodec-2-enyl, iso-Dodecenyl, n-Dodec-2-enyl oder n-Octadec-4-enyl.

C₁,-C₆-Alyklcarbonyl ist eine geradkettige oder verzweigte Carbonylgruppe mit einem Alkylrest mit ein bis sechs Kohlenstoffatomen, wie z.B. Acetyl, Propionyl, Butyryl, Isobutyryl, Valeryl, Isovaleryl, Pivalolyl und ähnliche.

Geeignete hydroxysubstituierte C₁-C₂₀-Alkylgruppen sind z.B. Hydroxymehtyl, Hydroxyethyl, Hydroxypropyl, Hydroxybutyl, Hydroxypentyl, Hydroxyhexyl, Hydroxyheptyl, Hydroxyoctyl, Hydroxynonyl, Hydroxydecyl und ahnliche.

Vorzugsweise werden Hydroxydiphenylether der Formel (1) verwendet, worin
- R₁,R₂ und R₃: unabhängig voneinander Wasserstoff; C₁-C₂₀-Alkyl; hydroxy-substituiertes C₁-C₂₀-Alkyl; oder C₁-C₂₀-Alkoxy;
- R₄: Wasserstoff; C₁-C₂₀-Alkyl; hydroxy-substituiertes C₁-C₂₀-Alkyl; Hydroxy; Formyl; Acetonyl; Allyl; Carboxymethyl; oder Carboxyallyl; und
- R₅: Wasserstoff; C₁-C₂₀-Alkoxy; oder C₁-C₆-Alkylcarbonyl;
wobei mindestens einer der Substituenten R₁, R₂, R₃ und R₄ nicht Wasserstoff bedeutet.

Ganz besonders bevorzugt sind Verbindungen der Formel (1), worin
- R₁: C₁-C₁₆-Alkyl; und
- R₂, R₃ R₄ und R₅: Wasserstoff;
bedeuten.

Vorzugsweise entspricht die Komponente (a) der Formel 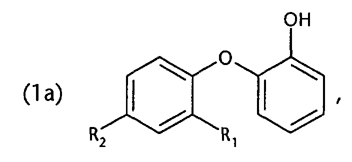 worin R₁ und R₂ unabhängig voneinander Wasserstoff; C₁-C₂₀-Alkyl; oder hydroxy-substituiertes C₁-C20-Alkyl; wobei mindestens einer der Substituenten R₁ und R₂ nicht Wasserstoff bedeutet, bedeuten.

Ganz besonders bevorzugt sind Hydroxydiphenylether der Formel 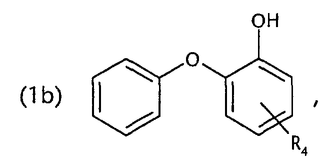 worin R₄ Carboxy; Carboxy-C₁-C₃-Alkyl; C₁-C₆-Alkylcarbonyl; oder C₁-C₃-Alkylcarbonyl-C₁-C₃-alkyl; bedeuten.

Weiterhin werden erfindungsgemäss Verbindungen der Formel 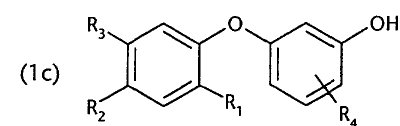 verwendet, worin
- R₁, R₂ und R₃: unabhängig voneinander Wasserstoff; Hydroxy; C₁-C₂₀-Alkyl; oder hydroxy-substituiertes C₁C₂₀-AIkyl; und
- R₄: C₁-C₂₀-Alkyl; hydroxysubstituiertes C₁-C₂₀-Alkyl; Phenyl; Phenyl-C₁-C₃-Alkyl; oder C₁-C₆-Alkylcarbonyl;
bedeuten; und insbesondere Verbindungen der Formel (1c), worin
R₁,R₂ und R₃ Wasserstoff; oder C₁-C₂₀-Alkyl; bedeuten.

Ganz besonders bevorzugt sind Verbindungen der Formel (1c), worin
- R₁, R₂ und R₃: Wasserstoff; und
- R₄: C₁-C₂₀-Alkyl; Phenyl-C₁-C₅-Alkyl; oder C₁-C₆-Alkylcarbonyl;
bedeuten.

Bevorzuqt werden auch Verbindunqen der Formel 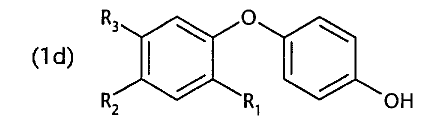 verwendet, worin
- R₁, R₂ und R₃: unabhängig voneinander Wasserstoff; C₁-C₂₀-Alkyl; hydroxy-substituiertes C₁-C₂₀-Alkyl; Cyclo-C₅-C₇-Alkyl; Phenyl-C₁-C₃-Alykl bedeuten, wobei mindestens einer der Substituenten R₁, R₂ oder R₃ nicht Wasserstoff bedeutet.

Ganz besonders bevorzugt sind Verwindungen der Formel (1d), worin
- R₁ und R₃: unabhängig voneinander C₁-C₂₀-Alkyl; und
- R₂: Wasserstoff
bedeuten.

Weitere erfindungsgemäss verwendbare Hydroxydiphenyletherverbindungen sind in der nachfolgenden Tabelle 1 aufgeführt:

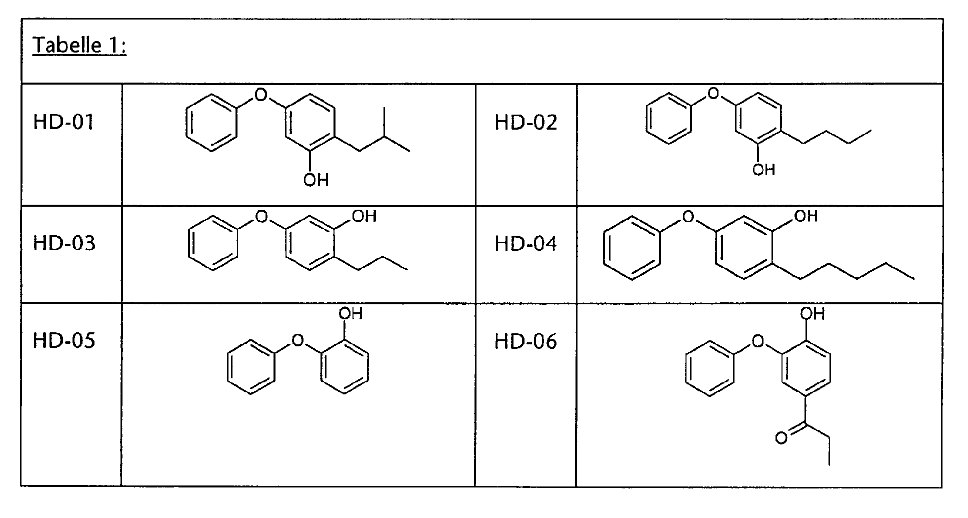

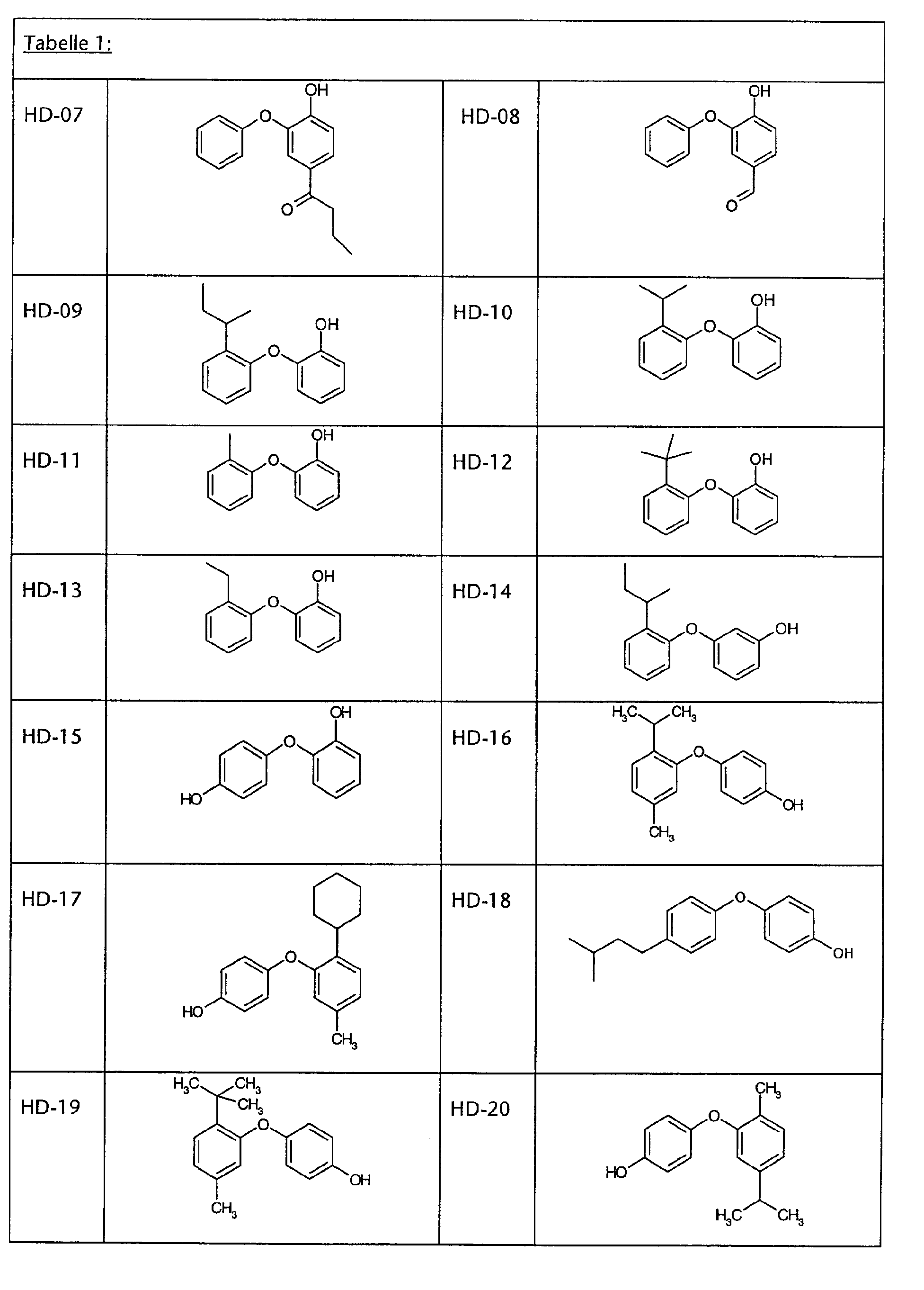

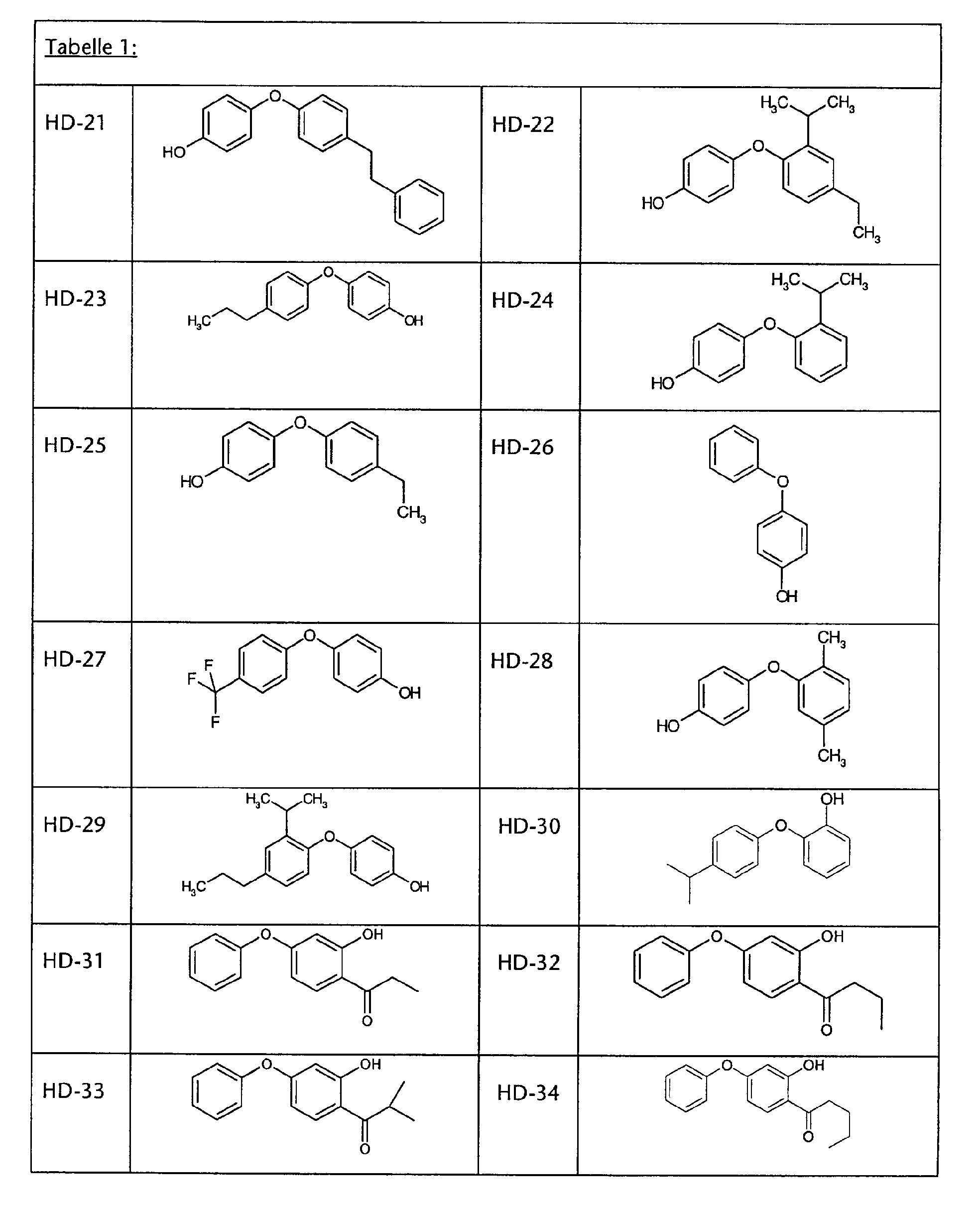

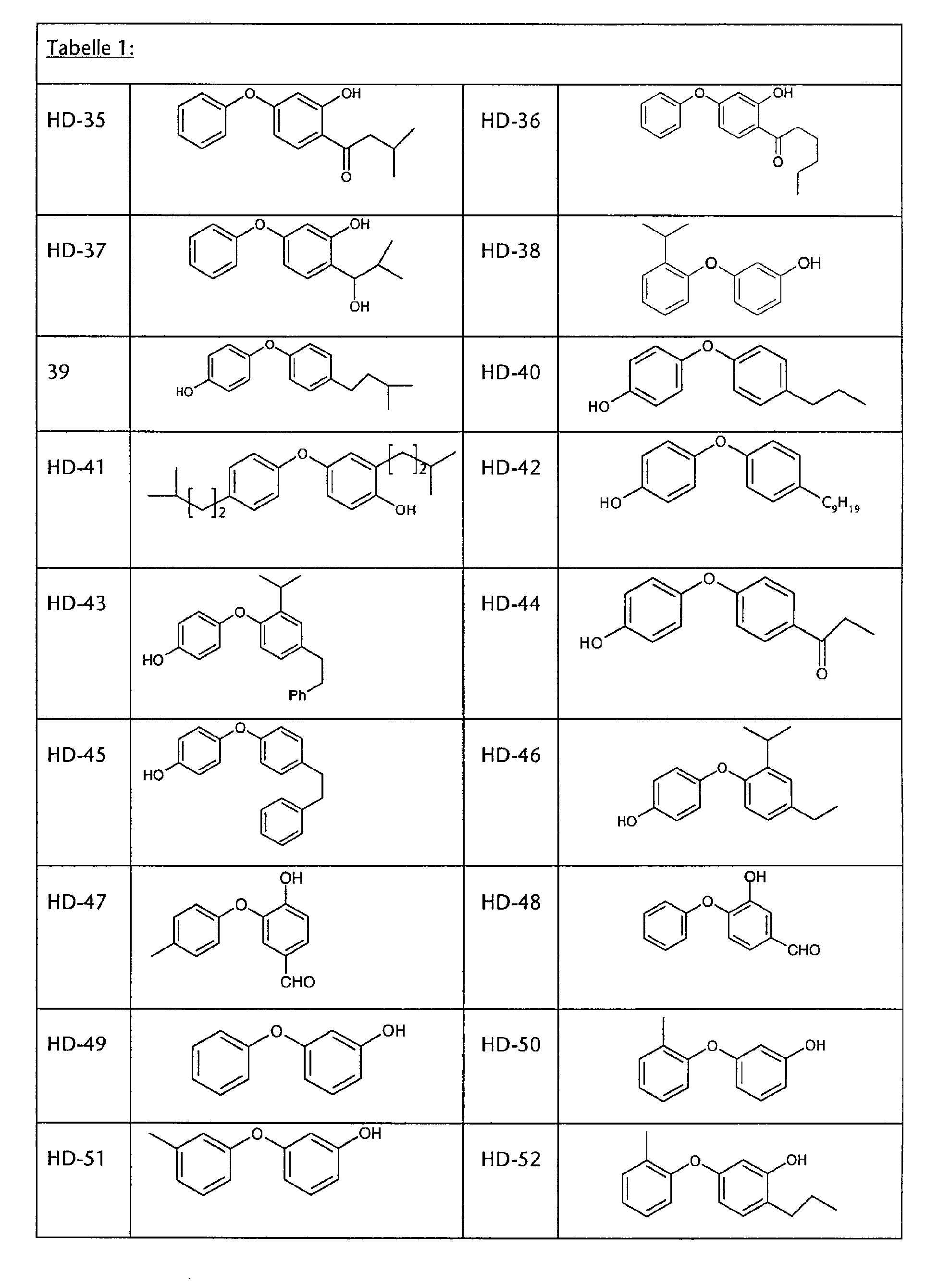

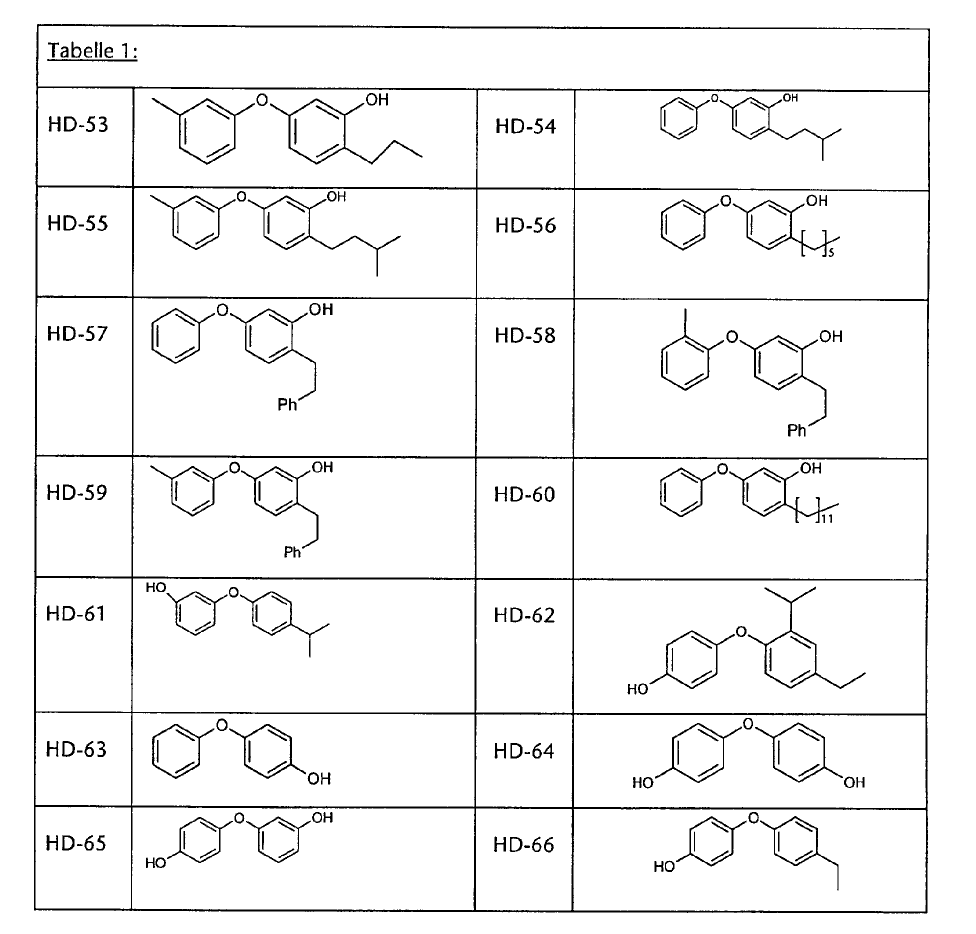

Neben den Hydroxydiphenylehterverbindungen der Komponente (a) können erfindungsgemäss zusätzlich weitere Substanzen mit hautaufhellenden Eigenschaften (Komponente (b)) verwendet werden.

Als Komponente (b) können z.B. die folgenden Substanzen oder Substanzklassen verwendet werden:
1. u-Pyronderivate, entsprechend Formel 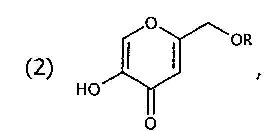 worin
   R Wasserstoff (= Kojisäure; 5-Hydroxy-2-hydroxymethyl-4H-pyran-4-on); oder den Rest der Formel (2a) 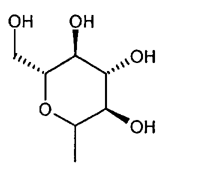 bedeutet, und deren Derivate, wie z.B. Kojisäureglucoside.
2. Hydrochinon auch als Glycoside und Hydrochinonderivate als Glycoside, wie z.B. 4-Hydroxyphenyl-D-glucopyranosid (= α-, bzw. β-Arbutin), entsprechend Formel 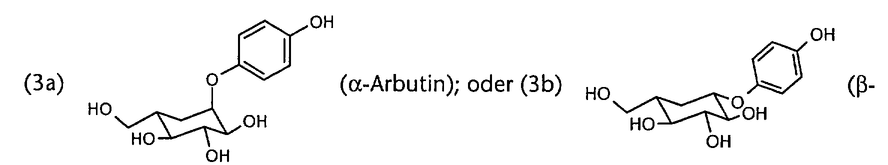 Arbutin); 4-Methoxyphenethylmethylether-D-glucopyranosid; 1,5,9,1 3-tetramethyl-4,8,12-tetradecatrienyl (9Cl); 5,9,13-Pentadecatrien-2-ol, 6,10,14-trimethyl-(9Cl); 1,5,9,13-tetramethyltetradecyl-D-glucopyranosid.
3. Resorcinderivate wie Glabridin (1,3-Benzenediol, 4-[(3R)-3,4-dihydro-8,8-dimethyl-2H,8H-benzo[1,2-b:3,4-b']dipyran-3-yl]-) oder 4-Butylresorcinol (=Rucinol); 2,4-Dihydroxybenzophenone und isomere Benzophenone;
4. Glycine, L-a-glutamyl-L-cysteinyl- (= Glutathion); Acetylcystein; Oligopeptide;
5. Alkyldicarbonsäuren, wie Azelainsäure (Nonandicarbonsäure) und ihre Mono- und Diester;
6. 1,2-Dihydroxyphenylderivate, wie z.B. 4-(3,4-Dihydroxyphenyl)butan-2-ol; 4-Hydroxy-3-methoxybenzylacetone (=Gingerone); 4H-1-Benzopyran-4-one, 2-(3,4-dihydroxyphenyl)-3,5,7-trihydroxy- (= Quercitin), entsprechend Formel 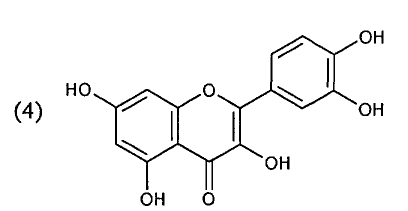
7. Urea, (2,5-dioxo-4-imidazolidinyl)- (= Allantoin), entsprechend Formel 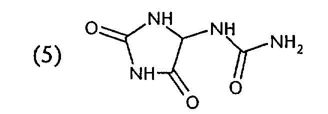
8. Furanone, wie 3-Hydroxy-4,5-dimethyl-2(5H)-furanon; 3-Hydroxy-4-methyl-5-ethyl-2(5H)-furanon;
9. Phenylacetaldehyde;
10. Benzaldehyde; wie z.B. 4-Hydroxybenzaldehyd und 3-Methylbenzaldehyd;
11. 4-Methoxycinnamaldehyde;
12. Isomere Decensäure (C₁₀H₁₈O₂);
13. Ascorbinsäure und Derivate, wie z.B. 6-Acylascorbinsäure-2-glucosid; Sulfate, Stearate oder Phospate der Ascorbinsäure;
14. Salicylsäurederivate, wie 6-[(8Z)-8-pentadecenyl]-salicylsäure; (Anacardinsäuremonoen) und 6-[(8Z, 11Z)-8, 11, 14-pentadecatrienyl] Salicylsäure (Anacardinsäuretriene);
15. Phenolsche Substanzen, wie 3-[8(Z)-Pentadecenyl]phenol oder Curuminphenolische Substanzen, wie z.B. Curcumin;
16. Benzo[b]pyranderivative, wie [1 ]Benzopyrano[5,4,3-cde][1 ]benzopyran-5,1 0-dion, 2,3,7,8-tetrahydroxy- (7Cl, 8Cl, 9Cl) (= Ellagic Säure); 2'-Hydroxy-2,4,4,7,4'-pentamethylflavan; 2'-Flavanol, 2,4,4,4',7-pentamethyl-, acetat; 2-(3,4-dihydro-2,4,4,7-tetramethyl-2H-1-benzopyran-2-yl)-5-methylphenyl und (8u-glglycopyranosyl-7-hydroxy-5-methyl-2-(2-oxopropyl)-4H-1-Benzopyran-4-on (Aloesin), entsprechend der Formel 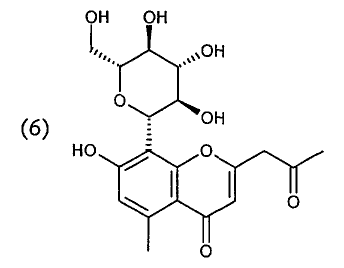
17. Bornyl- und Cinnamat-Derivate, wie 2-Propenoic acid, 3-(4-hydroxyphenyl)-, 1,7,7-trimethylbicyclo[2.2.1]hept-2-yl ester, endo-; 2-Propenoic acid, 3-(4-methoxyphenyl)-, 1,7,7-trimethylbicyclo[2.2.1]hept-2-yl ester, endo-; 2-Propenoic acid, 3-(4-hydroxyphenyl)-, 1-methyl-3-(2,2,6-trimethylcyclohexyl)propyl ester; 2-Propenoic acid, 3-phenyl-, 1-methyl-3-(2,2,6-trimethylcyclohexyl)propyl ester; 2-Propenoic acid, 3-[4-(u-D-glucopyranosyloxy)phenyl]-, (1 R,2S,4R)-1,7,7-trimethylbicyclo[2.2.1 ]hept-2-yl ester.
18. Azulene und deren Derivate, wie z.B. Guajazulen oder Vetivazulen, sowie Guaiol;
19. Zellbotenstoffe, wie Zytokine; Prostaglandine und Peptidwachstumsfaktoren;
20. α -Hydroxy-Carbonsäuren, wie z.B. die α -Hydroxypropionsäure (Milchsäure) sowie die Zitronen- und Aconitsäure
21. Verbindung der Formel 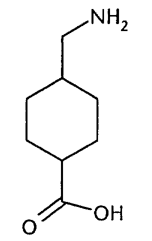
22. Verbindung der Formel

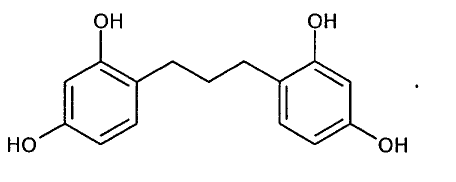

Vorzugsweise werden als Komponente (b) Kojisäure, α-Arbutin, Quercitin, Aloesin, Azelainsäure, Guaiol, Ellagsäure und deren Ester eingesetzt.

Jede der oben angegebenen Inhibitoren können als Einzelverbindungen oder als Gemische untereinander in der erfindungsgemässen Zusammensetzung verwendet werden.

Es können auch mehr als eine der zusätzlichen hautaufhellenden Verbindungen verwendet werden, wie z.B. zwei, drei oder vier weitere Verbindungen der Komponente (b).

Vorzugsweise ist das Verhältnis der Komponente (a) : (b) 1 : 99, vorzugsweise 5 :95, und insbesondere 10 : 90 bis 99 : 1, vorzugsweise 95 : 5, und insbesondere 90 : 10 Gew.-% der Komponente (b).

Insbesondere sind Mischungen aus (a) und (b) bevorzugt, worin das Verhältnis (a) : (b) von 20 : 80, vorzugsweise 30 : 70 bis 80 : 20, vorzugsweise 70 : 30 ist.

Die letztgenannten Zusammensetzungen können u.a. zur Verbesserung der Löslichkeit oder zur Erhöhung der die Haut aufhellenden Wirkung eingesetzt werden.

Weiterhin können die erfindungsgemäss verwendeten Zusammensetzungen als Komponente (c) einen oder mehrere UV-A- oder UV-B-Absorber der folgenden Substanzklassen enthalten:
1. p-Aminobenzoesäurederivate, wie z.B. 4-Dimethylaminobenzoesäure-2-ethylhexylester;
2. Salicylsäurederivate, wie z.B. Salicylsäure-2-ethylhexylester;
3. Benzophenonderivate, wie z.B. 2-Hydroxy-4-methoxybenzophenon und sein 5-sulfonsäurederivat;
4. Dibenzoylmethanderivate, wie z.B. 1 -(4-tert.-Butylphenyl)-3-(4-methoxyphenyl)propan-1,3-dion;
5. Diphenylacrylate, wie z.B. 2-Ethylhexyl-2-cyano-3,3-diphenyl acrylat und 3-(Benzofuranyl)-2-cyanoacrylat;
6. 3-lmidazol-4-yl-acrylsäure und -ester;
7. Benzofuranderivate, insbesondere 2-(p-Aminophenyl)benzofuranderivate, beschrieben in der EP-A-582,189, US-A-5,338,539, US-A-5,518,713 und der EP-A-613,893;
8. polymere UV-Absorber wie z.B. die in der EP-A-709,080 beschriebenen Benzylidenmalonatderivate;
9. Zimtsäurederivate, wie z.B. die in der US-A-5,601,811 und WO 97/00851 offenbarten 4-Methoxyzimtsäure-2-ethylhexylester bzw. lsoamylester oder Zimtsäurederivate;
10. Campherderivate, wie z.B. 3-(4'-Methyl)benzyliden-bornan-2-on, 3-Benzyliden-bornan-2-on, N-[2(und 4)-2-Oxyborn-3-yliden-methyl)-benzyl]acrylamid-Polymer, 3-(4'-Trimethylammonium)-benzyliden-bornan-2-on methylsulfat, 3,3'-(1,4-Phenylendimethin)-bis(7,7-dimethyl-2-oxo-bicyclo-[2.2.1]heptan-1-methansulphonsäure) und Salze, 3-(4'-Sulfo)benzyliden-bornan-2-on und Salze; Campherbenzalkoniummethosulfat;
11. Hydroxyphenyltriazinverbindungen, wie z.B. 2-(4'-Methoxyphenyl)-4,6-bis(2'-hydroxy-4'-n-octyloxyphenyl)-1,3,5-triazin; 2,4-Bis-{[4-(3-(2-propyloxy)-2-hydroxy-propyloxy)-2-hydroxy]-phenyl}-6-(4-methoxyphenyl)-1,3,5-triazin; 2,4-Bis-{[4-(2-ethyl-hexyloxy)-2-hydroxy]-phenyl}-6-[4-(2-methoxyethyl-carboxyl)-phenylamino]-1,3,5-triazin; 2,4-Bis-{[4-(tris(trimethylsiloxy-silylpropyloxy)-2-hydroxyj-phenyl}-6-(4-methoxyphenyl)-1,3,5-triazin; 2,4-Bis-([4-(2"methylpropenyloxy)-2-hydroxy]-phenyl}-6-(4-methoxyphenyl)-1,3,5-triazin; 2,4-Bis-{[4-(1 ',1 ',1 ',31,5',5',5'-Heptamethyltrisilyl-2"-methyl-propyloxy)-2-hydroxy]-phenyl}-6-(4-methoxyphenyl)-1,3,5-triazin; 2,4-Bis-{[4-(3-(2-propyloxy)-2-hydroxy-propyloxy)-2-hydroxy]-phenyl}-6-[4-ethylcarboxyl)-phenylamino]-1,3,5-triazin;
12. Benztriazolverbindungen, wie z.B. 2,2'-Methylen-bis-(6-(2H-benztriazol-2-yl)-4-(1,1,3,3-tetramethylbutyl)-phenol;
13. Trianilino-s-Triazinderivate, wie z.B. 2,4,6-Trianilin-(p-carbo-2'-ethyl-1'-oxi)-1,3,5-triazin sowie die in der US-A-5,332,568, EP-A-517,104, EP-A-507,691, WO 93/17002 und EP-A-570,838 offenbarten UV-Absorber;
14. 2-Phenylbenzimidazol-5-sulfonsäure und deren Salze;
15. Menthyl-o-aminobenzoat;
16. TiO₂ (unterschiedlich umhüllt), ZnO und Mica.

Auch die in "Sunscreens", Eds. N.J. Lowe, N.A.Shaath, Marcel Dekker, Inc., New York and Basel oder in Cosmetics & Toiletries (107), 50ff (1992) beschriebenen UV-Absorber können als zusätzliche UV-Schutzstoffe verwendet werden.

Besonders bevorzugt sind hierbei die in der folgenden Tabelle 2 angegebenen Lichtschutzmittel:

**Tabelle 2**

| INCl | Chemical Name | CAS No. |
|---|---|---|
| 3-BENZYLIDENE CAMPHOR | 1,7,7-trimethyl-3-(phenylmethylene)bicyclo[2.2.1 ]heptan-2-one | 15087-24-8 |
| 4-METHYLBENZYLIDENE E CAMPHOR | (+/-)-1,7,7-trimethyl-3-[(4-methylphenyl)methylene]bicyclo[2.2.1 ]heptan-2-one | 36861-47-9 |
| BENZOPHENONE-10 | (2-Hydroxy-4-methoxyphenyl)(4-methylphenyl)methanone | 1641-17-4 |
| BENZOPHENONE-1 | 2,4-dihydroxybenzophenone | 131-56-6 |
| BENZOPHENONE-2 | 2,2',4,4'-tetrahydroxybenzophenone | 131-55-5 |
| BENZOPHENONE-3 | 2-Hydroxy-4-methoxy benzophenone; | 131-57-7 |
| BENZOPHENONE-4 | 2-Hydroxy-4-methoxy benzophenone-5-sulfonic acid | 4065-45-6 |
| BENZOPHENONE-6 | 2,2'-dihydroxy-4,4'-dimethoxybenzophenone | 131-54-4 |
| BENZOPHENONE-8 | 2,2'-Dihydroxy-4-methoxybenzophenone | 131-53-3 |
| BENZYLIDENE CAMPHOR SULFONIC ACID | Alpha-(2-oxoborn-3-ylidene)toluene-4-sulphonic acid and its salts | 56039-58-8 |
| BUTYL METHOXYDIBENZOYLMETHANE | 1-[4-(1,1-dimethylethyl)phenyl]-3-(4-methoxyphenyl)propane-1,3-dione | 70356-09-1 |
| CAMPHOR BENZALKONIUM METHOSULFATE | Methyl N,N,N-trimethyl-4-[(4,7,7-trimethyl-3-oxobicyclo[2,2,1]hept-2-ylidene)methyl]anilinium sulphate; | 52793-97-2 |
| CINOXATE | 2-Ethoxyethyl-p-methoxycinnamate | 104-28-9 |
| DEAMETHOXYCINNAMATE | Diethanolamine salt of p-methoxy hydro cinnamate | 56265-46-4 |
| DIISOPROPYL METHYL CINNAMATE | 2-propenoic acid, 3-[2,4-bis(1-methylethyl)phenyl]-, methyl ester | 32580-71-5 |
| DIPROPYLENE GLYCOL SALICYLATE | Dipropylene glycol salicylate | 7491-14-7 |
| ETHYL DIHYDROXYPROPYL PABA | Ethyl 4-bis(2-hydroxypropyl)-aminobenzoate | 58882-17-0 |
| ETHYL DIISOPROPYLCINNAMATE | Ethyl 3- [2,4-bis(1-methylethyl)phenyl]acrylate | 32580-72-6 |
| ETHYL METHOXYCINNAMATE | Ethyl p-methoxycinnamate | 1929-30-2 |
| GLYCERYL OCTANOATE DIMETHOXYCINNAMATE | | |
| GLYCERYL PABA | Glyceryl 1- (4-aminobenzoate) | 136-44-7 |
| HOMOSALATE | 3,3,5-Trimethyl cyclohexyl-2-hydroxy benzoate | 118-56-9 |
| ISOAMYL p-METHOXYCINNAMATE | Isopentyl p-methoxycinnamate | 71617-10-2 |
| ISOPROPYL DIBENZOYLMETHANE | 1-[4-(1-methylethyl)phenyl]-3-phenylpropane-1,3-dione | 63250-25-9 |
| ISOPROPYL METHOXYCINNAMATE | Isopropyl p-methoxycinnamate | 5466-76-2 |
| LAWSONE | 2-Hydroxy-1,4-naphthoquinone | 83-72-7 |
| MENTHYL ANTHRANILATE | Menthyl-o-aminobenzoate | 134-09-8 |
| MENTHYL SALICYLATE | Menthyl salicylate | 89-46-3 |
| OCTOCRYLENE | 2-Ethylhexyl 2-cyano,3,3-diphenylacrylate | 6197-30-4 |
| ETHYLHEXYL DIMETHYL PABA | 2- ethylhexyl 4- (dimethylamino)benzoate | 21245-02-3 |
| ETHYLHEXYL METHOXYClNNAMATE | 2- ethylhexyl 4-methoxycinnamate | 5466-77-3 |
| ETHYLHEXYL SALICYLATE | 2- ethylhexyl salicylate | 118-60-5 |
| ETHYLHEXYL TRIAZONE | Benzoic acid, 4, 4', 4"- (1, 3, 5- triazine- 2, 4, 6- triyltriimino)tris-, tris(2- ethylhexyl) ester; 2,4,6-Trianilino-(p-carbo-2'-ethylhexyl-1 '-oxi)-1,3,5-triazine | 88122-99-0 |
| PABA | 4- aminobenzoic acid | 150-13-0 |
| PEG-25 PABA | Benzoic acid, 4-amino-, ethylester, polymer with oxirane | 113010-52-9 |
| PENTYL DIMETHYL PABA | amyl dimethyl PABA | 14779-78-3 |
| PHENYLBENZIMIDAZOLE SULFONIC ACID | 2- phenyl-1 H-benzimidazole-5-sulphonic acid | 27503-81-7 |
| POLYACRYLAMIDOMETHY L BENZYLIDENE CAMPHOR | | 113783-61-2 |
| TEA-SALICYLATE | Triethanolamine salicylate | 2174-16-5 |
| TEREPHTHALYLIDENE DICAMPHOR SULFONIC ACID | 3, 3'- (1, 4- phenylenedimethylene)bis[7,7-dimethyl- 2- oxo- bicyclo[2.2.1 ]heptane-1-methanesulfonic acid] | 90457-82-2 |
| TITANIUM DIOXIDE | Titanium dioxide | 13463-67-7 |
| DIGALLOYL TRIOLEATE | Digalloyl trioleate | 17048-39-4 |
| ZINC OXIDE | Zinc oxide | 1314-13-2 |
| METHYLENE BIS-BENZOTRIAZOLYL TETRAM ETHYLBUTYLPHENOL | 2,2'-Methylene-bis-[6-(2H-benzotriazol-2-yl)-4-(1,1,3,3-tetramethylbutyl)-phenol] | 103597-45-1 |
| BIS-ETHYLH EXYLOXYPHENOL METHOXYPHENYLTRIAZINE | 2,4-bis{[4-(2-ethylhexyloxy)-2-hydroxy]phenyl}-6-(4-methoxyphenyl)-(1,3,5)triazine | 187393-00-6 |
| BISIMIDAZYLATE | 1 H-Benzimidazole-4,6-disulfonic acid, 2,2'-(1,4-phenylene)bis-, disodium salt | 180898-37-7 |
| DIETHYLHEXYL BUTAMIDO TRIAZONE | Benzoic acid, 4,4'-[[6-[[4-[[(1,1-dimethylethyl)amino]carbonyl]phenyl]amino]1,3,5-triazine-2,4-diyl]diimino]bis-, bis(2-ethylhexyl)ester | 154702-15-5 |
| | Phenol, 2-(2H-benzotriazol-2-yl)-4-methyl-6-[2-methyl-3-[1 ,3,3,3-tetramethyl-1 -[(trimethylsilyl)oxy]disiloxanyl]propyl]- | 155633-54-8 |
| BENZYLIDENE MALONATE POLYSILOXANE | alpha-(trimethylsilyl)-omega-(trimethylsilyloxy)poly[oxy(dimethyl)silylene]-co-[oxy(methyl)(2-(p-[2,2-bis(ethoxycarbonyl)vinyl]phenoxy}-1-methyleneethyl)silylene]-co-[oxy(methyl)(2-{p-[2,2-bis(ethoxycarbonyl)vinyl]phenoxy}prop-1-enyl)silylene] | 207574-74-1 |
| | 2-(4-Diethylamino-2-hydroxybenzoyl)benzoic acid hexylester | 302776-68-7 |

Jedes der oben angegebenen Lichtschutzmittel, insbesondere die in der Tabelle 2 als bevorzugt angegebenen Lichtschutzmittel, können als Einzelverbindungen oder auch als Mischungen untereinander verwendet werden, wie z.B. zwei, drei, vier, fünf oder sechs der in der Tabelle angegebenen Lichtschutzmittel.

Vorzugsweise werden in der erfindungsgemäss verwendeten Zusammensetzung als Komponente (c) Triazin-UV-Absorber der Formel 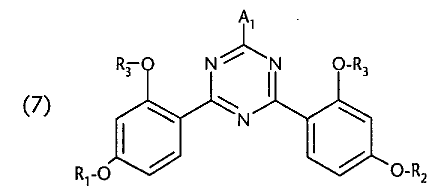 verwendet, worin
- R₁ und R₂,: unabhängig voneinander, C₃-C₁₈-Alkyl; C₂-C₁₈-Alkenyl; einen Rest der Formel -CH₂-CH(-OH)-CH₂-O-T₁; oder

- R₁ und R₂: einen Rest der Formel 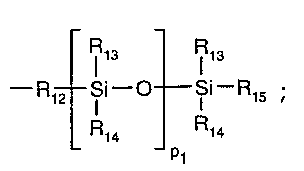
R₁₂ die direkte Bindung; einen geradkettigen oder verzweigten C₁-C₄-Alkylenrest oder einen Rest der Formel ―Cₘ₁H―2ₘ₁ oder ―Cm₁H2m₁―O― ;
R₁₃, R₁₄ und R₁₅, unabhängig voneinander, C₁-C₁₈-Alkyl; C₁-C₁₈-Alkoxy oder einen Rest der Formel 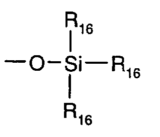
R₁₆ C₁-C₅-Alkyl;
m₁ und m₃, unabhängig voneinander, 1 bis 4;
p₁ 0 oder eine Zahl von 1 bis 5;
- A₁: einen Rest der Formel 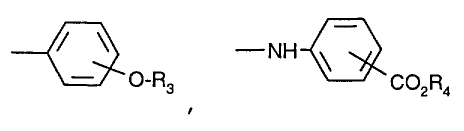 oder der Formel 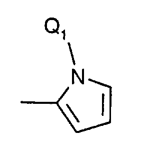
- R₃: Wasserstoff; C₁-C₁₀-Alkyl, -(CH₂CHR₅-O)_{n₁} -R4 ; oder einen Rest der Formel - CH₂-CH(-OH)-CH₂-O-T₁;
- R₄: Wasserstoff; M; C₁-C₅-Alkyl; oder einen Rest der Formel (CH₂)_{m₂} -O-T₁
- R₅: Wasserstoff; oder Methyl;
- T₁: Wasserstoff; oder C₁-C₈-Alkyl;
- Q₁: C₁-C₁₈-Alkyl;
- M: ein Metallkation;
- m₂: 1 bis 4; und
- n₁: 1-16;
bedeuten.

Besonders bevorzugt sind dabei die Verbindungen der Formeln 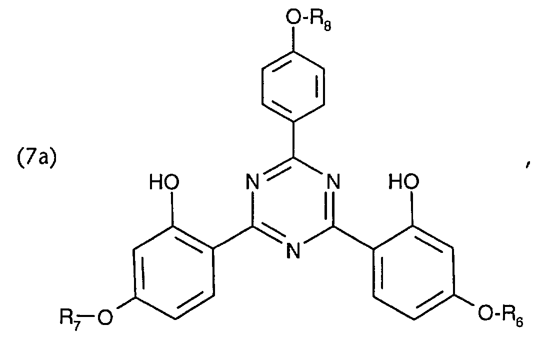 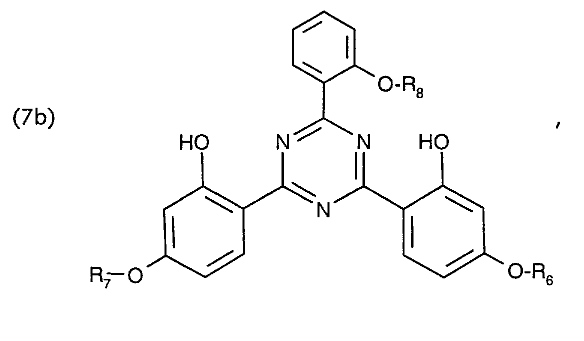 der Formel 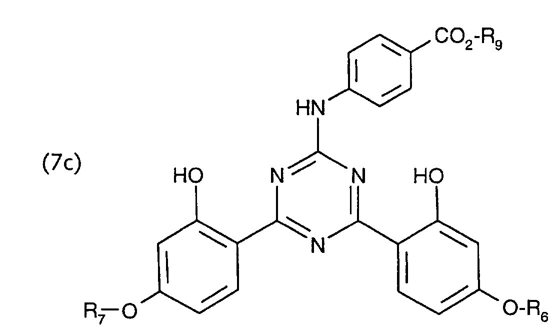 oder der Formel 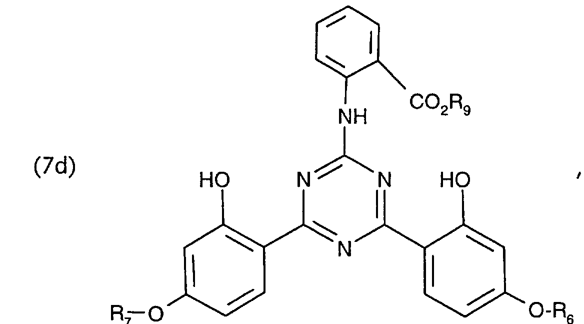 worin
- R₆ und R₇,: unabhängig voneinander, C₃-C₁₈-Alkyl; oder -CH₂-CH(-OH)-CH₂-O-T₁;
- R₈: C₁-C₁₀-AlkyI oder einen Rest der Formel 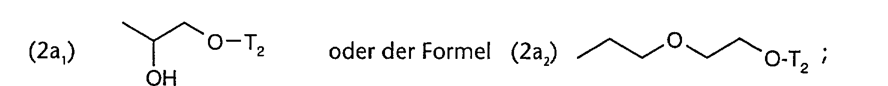
- R₉: Wasserstoff; M; C₁-C₅-Alkyl; oder einen Rest der Formel -(CH₂)ₘ-O-T₂;
- T₁ und T₂: unabhängig voneinander, Wasserstoff; oder C₁-C₅-Alkyl; und
- m: 1 bis 4;
bedeuten.

Im Vordergrund des Interesses stehen Verbindungen der Formel (7a) und (7b), worin R₆und R₇, unabhängig voneinander, C₃-C₁₈-Alkyl; oder -CH₂-CH(-OH)-CH₂-O-T,;
- R₈: C₁-C₁₀-Alkyl;
sowie Verbindungen der Formel (7a) und (7b), worin
- R₆ und R₇,: unabhängig voneinander, C₃-C₁₈-Alkyl oder -CH₂-CH(-OH)-CH₂-O-T₁; und
- T₁: Wasserstoff; oder C₁-C₅,-Alkyl;
bedeuten.

Als Beispiele für Verbindungen der Formel (7) seien genannt:
2-(4'-Methoxyphenyl)-4,6-bis(2'-hydroxy-4'-n-octyloxyphenyl)-1,3,5-triazin;
2,4-Bis-([4-(3-(2-propyloxy)-2-hydroxy-propyloxy)-2-hydroxy]-phenyl}-6-(4-methoxyphenyl)-1,3,5-triazin;
2,4-Bis-{[4-(2-ethyl-hexyloxy)-2-hydroxy]-phenyl}-6-[4-(2-methoxyethyl-carboxyl)-phenylamino]-1,3,5-triazin;
2,4-Bis-{[4-(tris(trimethylsiloxy-silylpropyloxy)-2-hydroxy]-phenyl}-6-(4-methoxyphenyl)-1,3,5-triazin;
2,4-Bis-{[4-(2"methylpropenyloxy)-2-hydroxy]-phenyl}-6-(4-methoxyphenyl)-1,3,5-triazin;
2,4-Bis-{[4-(1 ',1 ',1 ',3 ',5',5 ',5 '-Heptamethyltrisilyl-2 "-methyl-propyloxy)-2-hydroxy]-phenyl}-6-(4-methoxyphenyl)-1,3,5-triazin;
2,4-Bis-{[4-(3-(2-propyloxy)-2-hydroxy-propyloxy)-2-hydroxy]-phenyl)-6-[4-ethylcarboxyl)-phenylamino]-1,3,5-triazin; oder
2,4-Bis-([4-(2-ethyl-hexyloxy)-2-hydroxy]-phenyl}-6-(1-methylpyrrol-2-yl)-1,3,5-triazin.

Ganz besonders bevorzugt sind dabei solche Triazinverbindungen der Formel (2) bei denen R₆ und R₇die gleiche Bedeutung haben und insbesondere die Verbindung der Formel 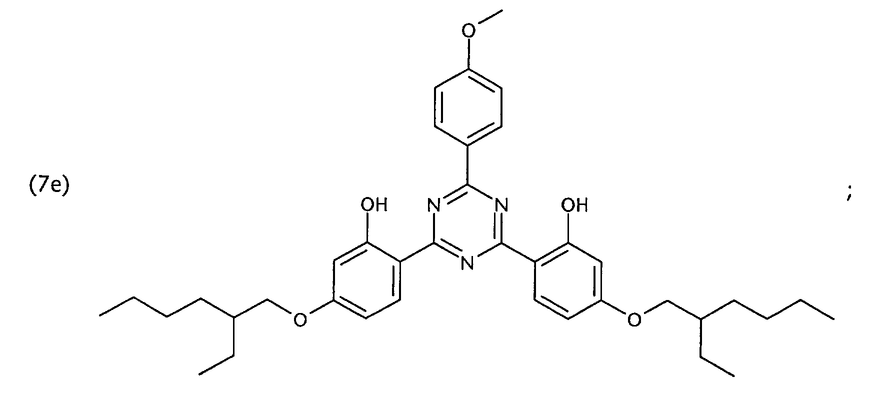 oder

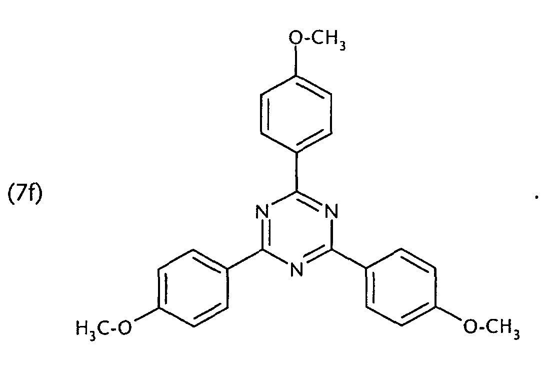

Weiterhin werden erfindungsgemäss als organische UV Absorber Benztriazolverbindungen der Formel 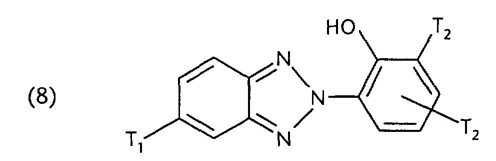 eingesetzt, worin
- T₁: Wasserstoff; oder C₁-C₃alkyl und
- T₂: nicht substituiertes C₁-C₄alkyl, vorzugsweise t-Butyl, oder mit Phenyl substituiertes C₁-C₄alkyl, vorzugsweise α,α-Dimethylbenzyl.

Bevorzugte, erfindungsgemäss eingesetzte Benztriazolverbindungen entsprechen der Formel 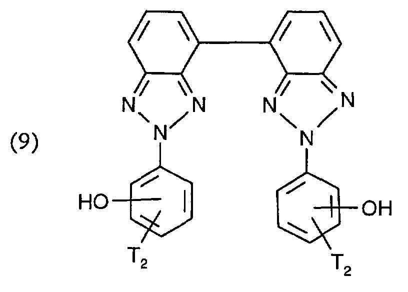 worin
- T₂: Wasserstoff; oder C₁-C₁₂-Alkyl, vorzugsweise iso-Octyl bedeutet.

Weiterhin werden bevorzugt Benztriazolverbindungen der Formel 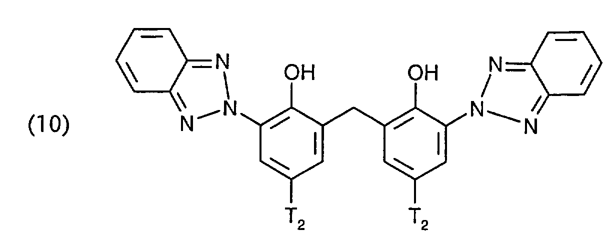 eingesetzt, worin
- T₂: C₁-C₁₂alkyl, vorzugsweise iso-Octyl bedeutet,

Weitere bevorzugte, erfindungsgemäss eingesetzte Verbindungen entsprechend Komponente (c) sind Octylmethoxycinnamat oder Benzophenon 3.

Neben den Komponenten (a), (b) und (c) können in der erfindungsgemässen Zusammensetzung als Komponente (d) auch sekundäre Lichtschutzmittel vom Typ der Antioxidantien eingesetzt werden, die die photochemische Reaktionskette unterbrechen, welche ausgelöst wird, wenn UV-Strahlung in die Haut bzw. Haare eindringt. Diese Eigenschaft ist beim kosmetischen Lichtschutz erwünscht, da unter UV- und Lichteinfluss sowohl in Formulierungen als auch auf der Haut schädliche Radikale gebildet werden können. Durch Ausrüstung der erfindungsgemässen Zusammensetzungen mit Antioxidantien wird neben dem Schutz vor UV-Schäden gleichzeitig ein Schutz gegen den photochemischen Abbau von Bestandteilen in der Formulierung erreicht. Typische Beispiele für solche Antioxidantien sind:
- Aminosäuren (z.B. Glycin, Histidin, Tyrosin, Tryptophan) und deren Derivate,
- Imidazole (z.B. Urocaninsäure) und deren Derivate,
- Peptide wie D,L-Carnosin, D-Carnosin, L-Carnosin und deren Derivate (z.B. Anserin),
- Carotinoide, Carotine (z.B. u-Carotin, u-Carotin, Lycopin) und deren Derivate,
- Chlorogensäure und deren Derivate,
- Liponsäure und deren Derivate (z.B. Dihydroliponsäure),
- Aurothioglycose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, u-Linoleyl-, Cholesteryl- und Glycerylester) sowie deren Salze,
- Dilaurylthiodipropionat, Distearylthiodipropionat, Thiodipropionsäure und deren Derivate (Ester, Ether, Peptide, Lipide, Nukleotide, Nukleoside und Salze) sowie
- Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen, ferner
- (Metall)-Chelatoren (z.B. u-Hydroxyfettsäuren, Palmitinsäure, Phytinsäure, Lactoferrin), u-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. u-Linolensäure, Linolsäure, Ölsäure), Folsäure und deren Derivate, Ubichinon und Ubichinol und deren Derivate, Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, u-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄), Selen und dessen Derivate (z.B. Selen-Methionin), Stilbene und deren Derivate (z.B. Stilbenoxid, trans-Stilbenoxid) und die erfindungsgemäss geeigneten Derivate (Salze, Ester, Ether, Zucker, Nukleotide, Nukleoside, Peptide und Lipide) dieser genannten Wirkstoffe.

Ferner seien die in untenstehender Tabelle aufgeführten phenolischen Antioxidantien genannt.

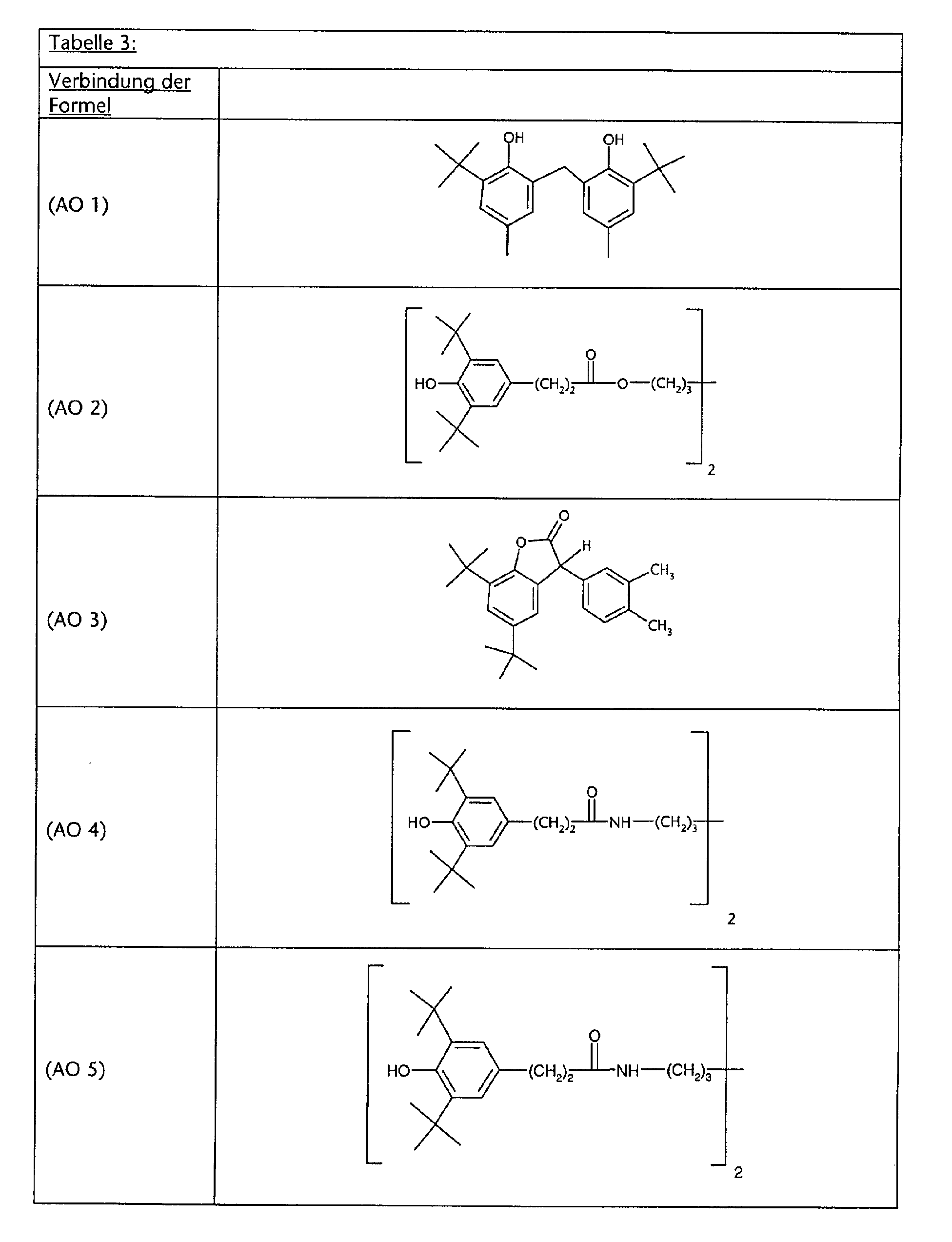

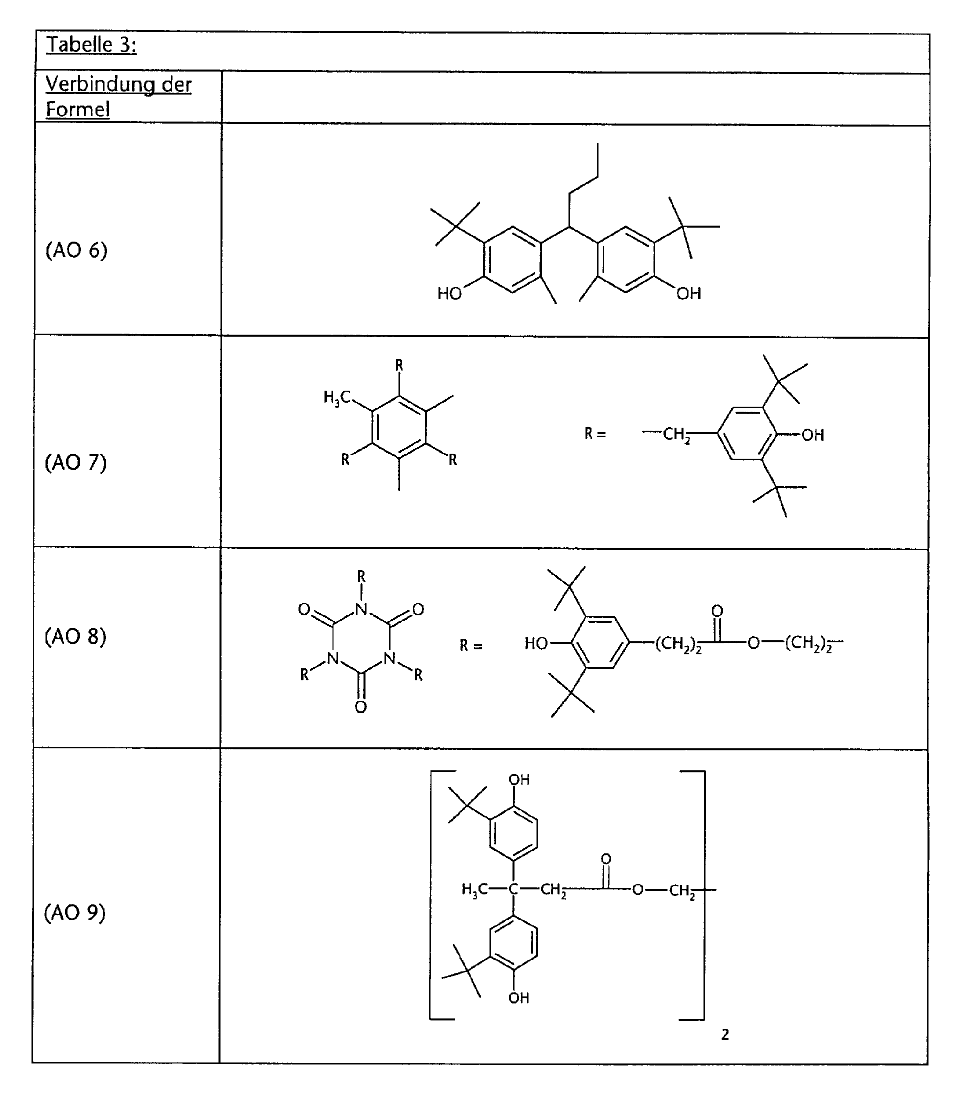

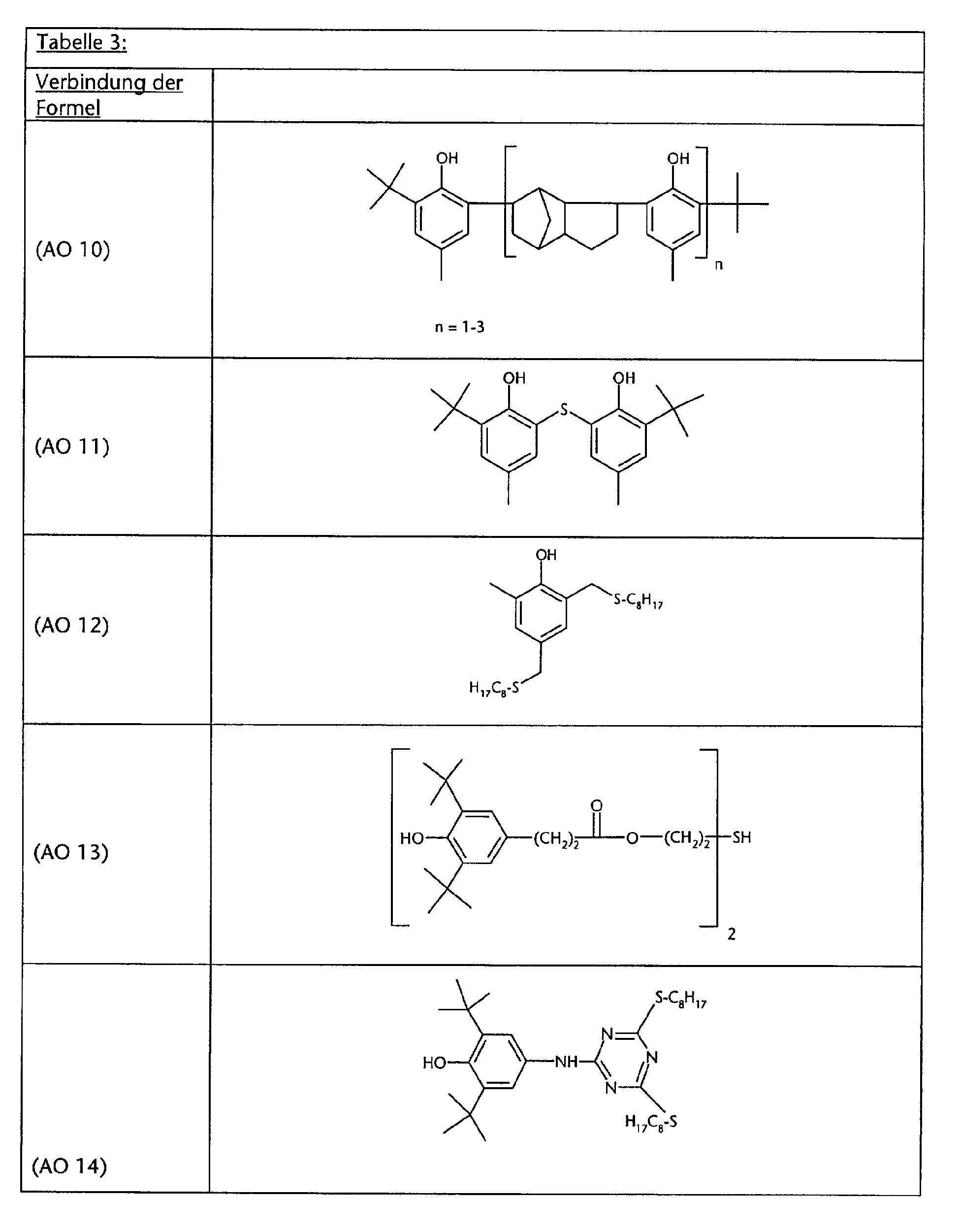

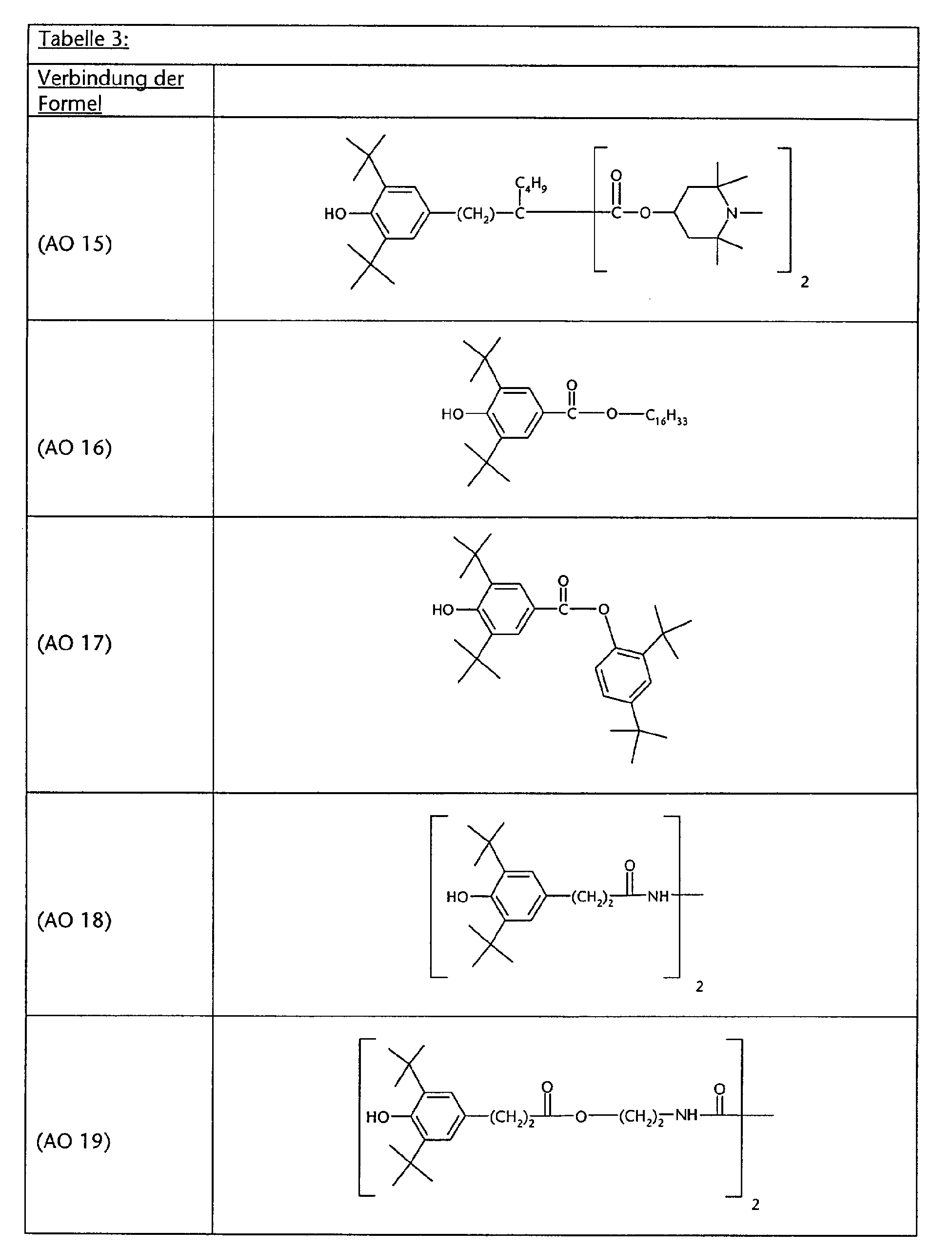

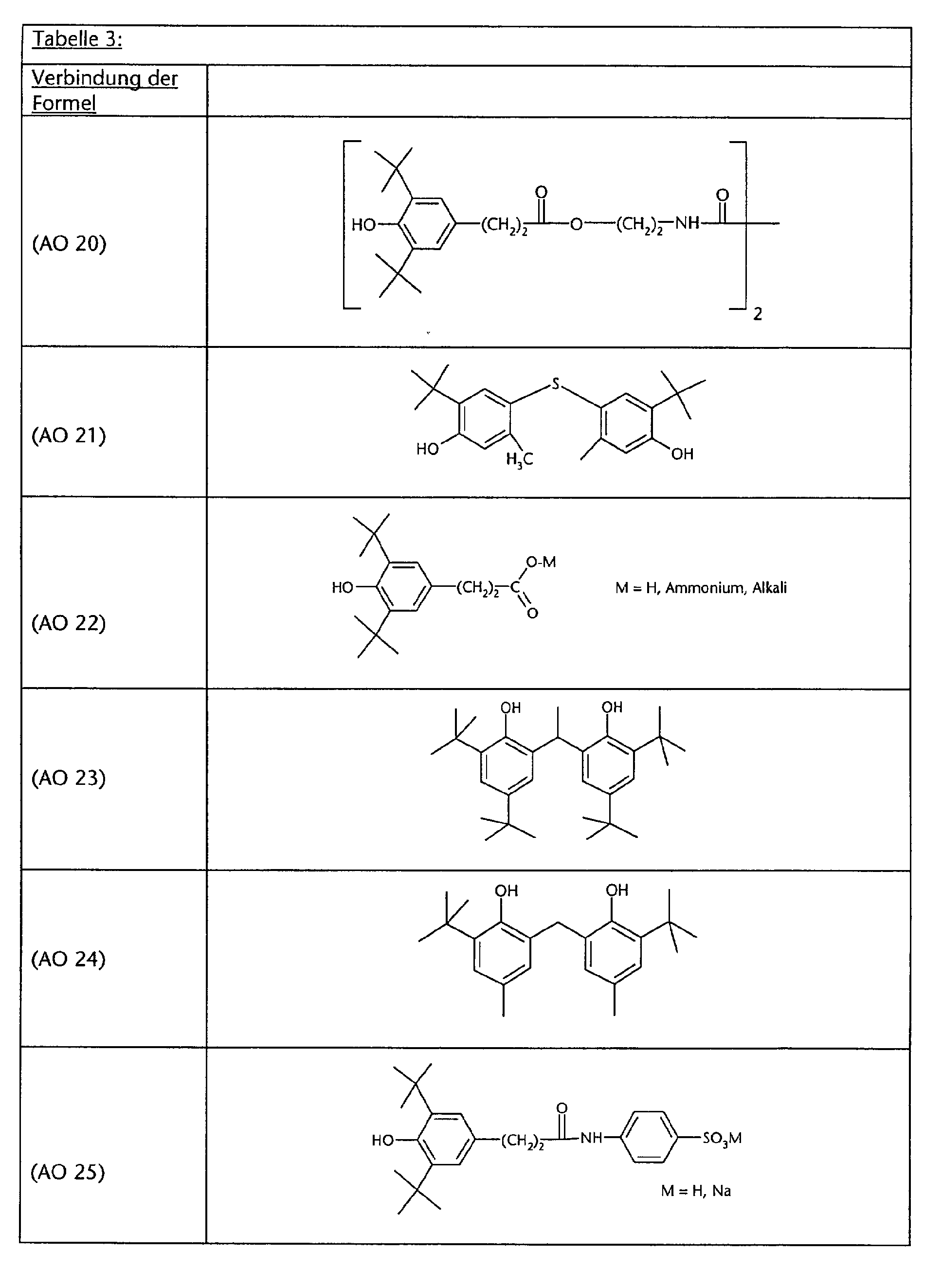

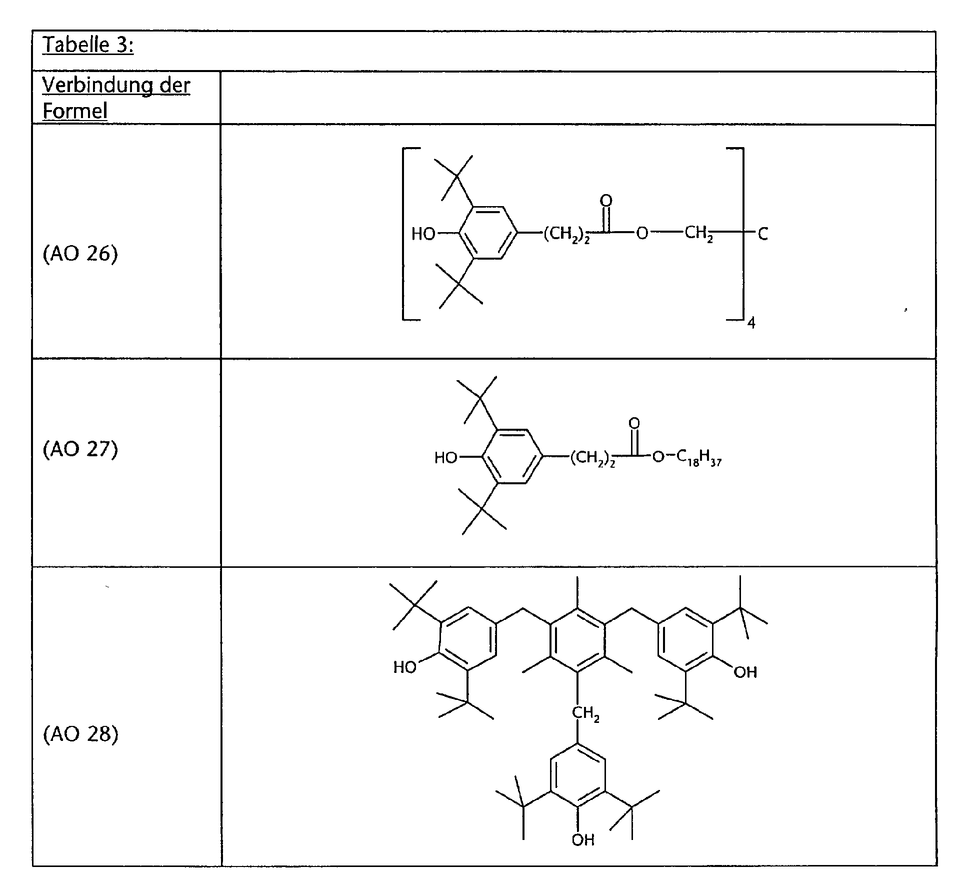

Ferner kommen für die erfindungsgemässe Zusammensetzung auch sterisch gehinderte Amine (auch HALS-Verbindungen (="Hindered Amine Light Stabilizers") genannt)) in Betracht.

Vorzugsweise handelt es sich dabei um ein 2,2,6,6-Tetraalkylpiperidinderivate.

Beispiele für erfindungsgemäss einsetzbare Tetraalkylpiperidinderivate sind der EP-A-356677, Seiten 3-17, Abschnitte a) bis f) zu entnehmen. Die genannten Abschnitte dieser EP-A werden als Teil der vorliegenden Beschreibung betrachtet. Besonders zweckmäßig setzt man folgende Tetraalkylpiperidinderivate ein:
Bis(2,2,6,6-tetramethyl-piperidin-4-yl)-sebacat, Bis(2,2,6,6-tetramethyl-piperidin-4-yl)-succinat, Bis(1,2,2,6,6-pentamethylpiperidin-4-yl)-sebacat, Bis(1 -octyloxy-2,2,6,6-tetramethylpiperidin-4-yl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)-nitrilotriacetat, Tetrakis(2,2,6,6-tetramethyl-4-piperidyl)-l,2,3,4-butantetraoat, 1,1 '-(1,2-Ethandiyl)-bis(3,3,5,5-tetramethyl-piperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis(1 -octyloxy-2,2,6,6-tetramethylpiperidyl)-sebacat, Bis(1-octyloxy-2,2,6,6-tetramethylpiperidyl)-succinat, Kondensationsprodukt aus N,N-Bis(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-nbutylamino-2,2,6,6-tetramethylpiperidyl)-1,3,5-triazin und 1,2-Bis(3-aminopropylamino)-ethan, Kondensationsprodukt aus 2-Chlor-4,6-di-(4-n-butylamino-1,2,2,6,6-pentamethylpiperidyl)-1,3,5-triazin und 1,2-Bis(3-aminopropylamino)-ethan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion, Gemisch von 4-Hexadecyloxy- und 4-Stearyloxy-2,2,6,6-tetramethylpiperidin, -2,6-dichlor-13,5-triazin, Kondensationsprodukt aus 1,2-Bis(3-aminopropylamino)-ethan und 2,4,6-trichlor-1,3,5-triazin sowie 4-Butylamino-2,2,6,6-tetramethyl-piperidin (CAS Reg. No. [136504-96-6]); (2,2,6,6-tetramethyl-4-piperidyl)-n-dodecylsuccinimid, (1,2,2,6,6-pentamethyl-4-piperidyl)-n-dodecylsuccinimid, 2-Undecyl-7,7,9,9-tetramethyl-1-oxa-3,8-diaza-4-oxo-spiro[4,5]decan. Umsetzungsprodukt von 7,7,9,9-Tetramethyl-2-cycloundecyl-1 -oxa-3,8-diaza-4-oxospiro[4,5]decan und Epichlorhydrin, Tetra(2,2,6,6-tetramethylpiperidin-4-yl)-butan-1,2,3,4-tetrac arboxylat, Tetra(1,2,2,6,6-pentamethylpiperidin-4-yl)-butan-1,2,3,4-tetracarboxylat, 2,2,4,4-Tetramethyl-7-oxa-3,20-diaza-21-oxo-dispiro[5.1.11.2] -heneicosan, 8-Acetyl-3-dodecyl-1,3,8-triaza-7,7,9,9-tetramethylspiro[4,5] -decan-2,4-dion, oder eine

Verbindung der Formel 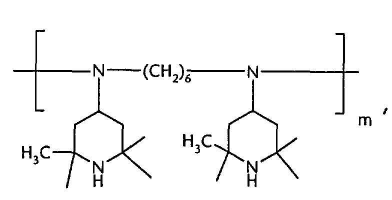 wobei m ein Wert von 5-50 bedeutet, 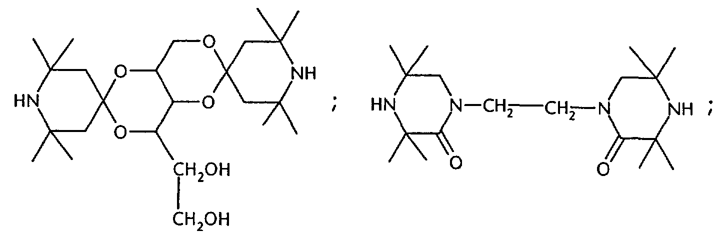 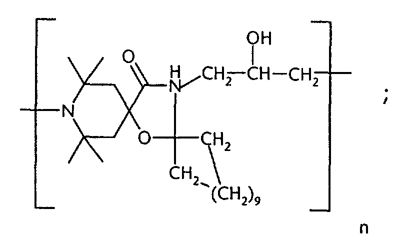 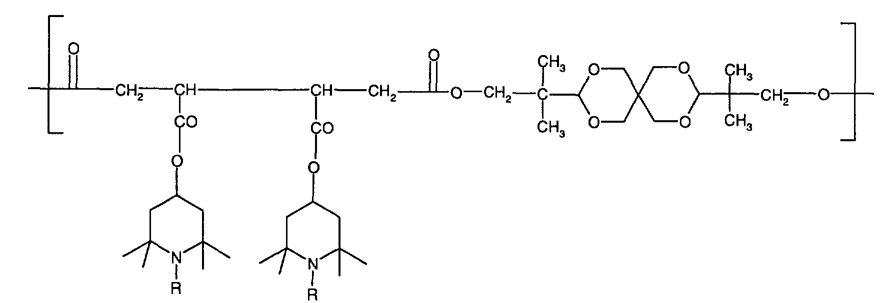 R = H oder CH₃ oder 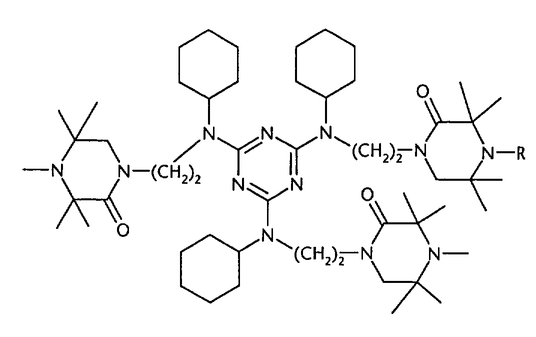 R = H oder CH₃

Der Anteil der Antioxidantien liegt dabei gewöhnlich zwischen 0,001 und 30, vorzugsweise 0,01 bis 3 Gew.-%, bezogen auf das Gewicht der erfindungsgemässen Zusammensetzung.

Die als zusätzliche Substanzen der Komponente (b), (c) bzw. (d) können verschiedene Funktionen haben:
a. direkter Einfluss auf die Melaninsynthese, wie z.B. Pyronderivate, insbesondere Kojisäure, 4-Hydroxyphenyl-D-glucopyranosid (α- bzw. β-Arbutin), Resorcinderivate, wie z.B. Resorcin, Benzo[b]pyranderivative, wie z.B. 8u-Glycopyranosyl-7-hydroxy-5-methyl-2-(2-oxopropyl)-4H-1-Benzopyran-4-on (Aloesin) oder Ellagsäure, Azulen, Guaiol und Vitamin C.
b. indirekter Einfluss auf die Melaninsynthese via interagierende Zellen, wie z.B Vitamin A, Zytokine, Prostaglandine oder Wachstumsfaktoren;
c. indirekter Einfluss durch mechanisches Abschälen der Hornhaut, wir z.B Salicylsäure oder Lactate, wie z.B. Milchsäure
d. indirekter Einfluss durch UV-Schutz: UV-A-Filter

In der untenstehenden Tabelle 3 sind Beispiele von Mischungen von Hydroxydiphenyletherverbindungen der Formel (1) (Komponente (a)) mit weiteren die Haut aufhellenden Wirksubstanzen (Komponente (b)) und UV-Absorbern (Komponente (c)) zusammengestellt. Die Angaben beziehen sich auf Gew.-% in der kosmetischen Endformulierung.

**Tabelle 3**

| Formulierung | | | | | | | | | | | |
|---|---|---|---|---|---|---|---|---|---|---|---|
| Komponente | C1 | C2 | C3 | C4 | C5 | C6 | C7 | C8 | C9 | C10 | C11 |
| Komponente (a) (Summe) | 0,05 | 0,1 | 0,1 | 0,1 | 0,2 | 0,3 | 0,3 | 0,3 | 0,5 | 1 | |
| (a₁) | 0,05 | 0,1 | 0,1 | 0,12 | 0,2 | 0,3 | 0,3 | 0,3 | 0,5 | 1 | 0 |
| Kompo nente (b) (Summe) | 0,1 | 0,2 | 0,4 | 0,2 | 0,1 | 0,2 | 0,3 | 0,5 | 0,3 | 5 | 5 |
| (b₁) | 0,05 | 0,1 | 0,2 | | 0,1 | | | 0,2 | 0,3 | | |
| (b₂) | 0,05 | | 0,1 | 0,2 | | 0,1 | | 0,3 | | 2 | 2 |
| (b₃) | | 0,1 1 | | | | | 0,1 | | | | |
| (b₄) | | | 0,1 | | | 0,1 | | | | | |
| (b₅) | | 0.5 | | | | | | 0, 3 | | | |
| (b₆) | | | | | | | | 0,2 | | | |
| (b₇) | | | 0,5 | | | | | | | | |
| (b₈) | | 2 | | | | | | | | | |
| (b₉) | 0,05 | | 0,1 | | | | | | | | |
| (b₁₀) | | | | | 0,1 | | | | | | |
| (b₁₁) | | | | | | | 0,2 | | | 3 | 3 |
| Komponente (c) (Summe) | 30 | 25 | 15 | 25 | 20 | 0 | 35 | 15 | 20 | 10 | 10 |
| (C₁) | 5 | | | | 5 | | | | | | |
| (C₂) | 2 | | | | | | 5 | | | 2 | 2 |
| (c₃) | | 10 | 5 | | | | | | | | |
| (c₄) | 10 | | | | 2 | | | | | | |
| (C₅) | 5 | | | | | | 5 | | | | |
| (C₆) | | | | | | | | | 5 | | |
| (C₇) | | | 5 | 10 | | | | 5 | | | |
| (C₈) | | | 5 | 5 | | | | | | | |
| (C₉) | | | | 5 | | | 5 | 5 | | | |
| (C₁₁) | | | | | 8 | | | | | | |
| (C₁₂) | | | | | | | 5 | | | | |
| (c₁₃) | | 5 | | | | | | | | | |
| (c₁₄) | | 5 | | 5 | | | 8 | | | | |
| (c₁₅) | | | | | 5 | | | | | | |
| (c₁₆) | | | | | | | 2 | | 2 | | |
| (c₁₇) | 3 | | | | | | | 5 | | | |
| (C₁₈) | 2 | | | | | | 5 | | | | |
| (C₁₉) | 2 | | | | | | | | 3 | | |
| (C₂₀) | | 3 | | | | | | | | 3 | 3 |
| (C₂₀) | | 2 | | | | | | | 5 | | |
| (C₂₁) | | | | | | | | | 2 | | |
| (c₂₂) | | | | | | | | | 3 | | |
| (c₂₃) | | | | | | | | | | 5 | 5 |
| Komponente (d) (Summe) | 1,1 | | 0,3 | | 0,1 | 0,2 | | | | 0,3 | 0,3 |
| (d₁) | 0,1 | | | | 0,1 | | | | | 0,1 | 0,1 |
| (d₂) | 1 | | | | | | | | | | |
| (d₃) | | | 0,3 | | | 0,2 | | | | 0,2 | 0,2 |

| | |
|---|---|
| (a₁) | Hydroxydiphenyletherverbindung der Formel (1) |
| (b₁) | 5-HYDROXY-2-HYDROXY-METHYL-4H-PYRAN-4-ONE (= KOJlC SÄURE) [501-30-4] |
| (b₂) | 4-HYDROXYPHENYL β-D-GLU-COPYRANOSIDE (= ARBUTIN) [497-76-7] |
| (b₃) | (2,5-DIOXO-4-IMIDAZOLI-DINYL)- UREA (= ALLAN-TOIN) [97-59-6] |
| (b₄) | 2-(3,4-DIHYDROXYPHE-NYL)-3,5,7-TRIHYDROXY-4H-1-BENZOPYRAN-4-ONE (= QUERCITIN) [117-39-5] |
| (b₅) | GUAIOL |
| (b₆) | AZULEN |
| (b₇) | SALICYLSÄURE |
| (b₈) | MILCHSÄURE |
| (b₉) | RESORCIN |
| (b₁₀) | ALOESIN |
| (b₁₁) | 1,7-HEPTANEDICARB-OXYLIC ACID (=AZELAIC ACID) [123-99-9] |
| (C₁) | BENZENESULFONIC ACID, 3-(2H-BENZOTRIAZOL-2-YL)-4-HYDROXY-5-(1 -METHYLPROPYL)-, MONOSODIUM SALT [92484-48-5] |
| (c₂) | PROPYL GALLATE [121-79-9] |
| (C₃) | N-[3-(3,5-DI-TERT-BUTYL-4-HYDROXYPHENYL)PROPIONYL] SULFANILC ACID (OR SALTS E.G. WITH SODIUM) |
| (c₄) | BENZYLIDENE MALONATE POLYSILOXANE [207574-74-1] |
| (C₅) | DROMETRIZOLE TRISILOXANE [155633-54-8] (15) |
| (C₆) | DIETHYLHEXYL BUTAMIDO TRIAZONE [154702-15-5] (10) |
| (C₇) | PHENOL, 2,2'-[6-(4-METHOXY-PHENYL)-1,3,5-TRIAZINE-2,4-DIYL]BIS[5-[(2-ETHYLHEXYL)OXY]- [187393-00-6] (10) |
| (C₈) | 1 H-BENZIMIDAZOLE-4,6-DISULFONIC ACID, 2,2'-(1,4-PHENYLENE)BIS-, DISODIUM SALT [180898-37-7] (10) |
| (C₉) | BIS-BENZOTRIAZOLYL TETRA-METHYLBUTYL-PHENOL [103597-45-1 (10) |
| (C₁₀) | TEREPHTHALYLIDENE (C10)DICAMPHOR SULFONIC ACID [90457-82-2] (10) |
| (C₁₁) | POLYACRYLAMIDOMETHYL BENZYLIDENE CAMPHOR [113783-61-2] (6) |
| (C₁₂) | PHENYLBENZIMIDAZOLE SULFONIC ACID [27503-81-7] (8) |
| (C₁₃) | ETHYLHEXYL METHOXYCINNA-MATE [5466-77-3] (10) |
| (C₁₄) | OCTOCRYLENE [6197-30-4] (10) |
| (C₁₅ | CAMPHOR BENZALKONIUM METHOSULFATE [52793-97-2] (6) |
| (C₁₆ | BUTYL METHOXYDIBENZOYL-METHANE [70356-09-1] (5) |
| (C₁₇) | BENZOPHENONE-3 [131 -57-7](10) |
| (C₁₈) | BENZOPHENONE-4 [4065-45-6](5) |
| (C₁₉) | 1-DODECANAMINIUM, N-[3-[[4-(DIMETHYLAMINO)-BENZOYL]AMINO]PROPYL]-N,N-DIMETHYL-, SALT WITH 4-METHYL-BENZENE-SULFONIC ACID [156679-41-3] |
| (C₂₀) | 1-PROPANAMINIUM, N,N,N-TRIMETHYL-3-[(1-OXO-3-PHENYL-2-PROPENYL)AMINO] CHLORIDE [177190-98-6] |
| (C₂₁) | 3-BENZYLIDENE CAMPHOR [15087-24-8] (2) |
| (C₂₂) | 4-METHYLBENZYLIDENE CAMPHOR [36861-47-9] (4) |
| (C₂₃) | BENZYLIDENE CAMPHOR SULFONIC ACID [56039-58-8] (6) |
| (d₁) | Vitamin A |
| (d₂) | Vitamin C |
| (d₃) | Vitamin C palmitat |

Die erfindungsgemässen Hydroxydiphenyletherverbindungen bzw. Mischungen dieser Verbindungen mit einem oder mehreren Vertretern der Komponente (b) und/oder (c) lassen sich als kosmetische Formulierungen einsetzen.

Ein weiterer Erfindungsgegenstand stellt daher eine kosmetische Formulierung dar. Sie ist dadurch gekennzeichnet, dass sie
(a) eine Verbindung der Formel (1); und gegebenenfalls
(b) weitere die Haut aufhellenden Wirksubstanzen, und gegebenenfalls
(c) einen oder mehrere UV-A- und/oder UV-B Absorber, und gegebenenfalls
(d) ein Antioxidans, sowie
kosmetisch verträgliche Hilfs- oder Trägerstoffe enthält.

Vorzugsweise enthält die erfindungsgemässe kosmetische Formulierung
0.001 bis 10, vorzugsweise 0,05 bis 1 Gew.-% der Komponente (a),
0 bis 10, vorzugsweise 0,05 bis 1 Gew.-% der Komponente (b),
0 bis 30 Gew.-%, vorzugsweise 0,1 bis 15 Gew.-% der Komponente (c) und
0 bis 30 Gew.-%, vorzugsweise 0,1 bis 15 Gew.-% der Komponente (d).

Gegebenenfalls liegen eine oder mehrere Verbindungen der Komponenten (a) bis (d) in speziellen Trägern vor, wie z.B. Nanotopes, Liposomen, Glaspartikel oder Nanokolloide.

Die erfindungsgemässen kosmetischen Formulierungen eignen sich zum Schützen von ultraviolett empfindlichen organischen Materialien, insbesondere der Haut, und zur Aufhellung der Haut.

Die erfindungsgemässen Zusammensetzungen können sowohl gelöst als auch im mikronisierten Zustand verwendet werden.

Die Herstellung der kosmetischen Zusammensetzungen kann durch physikalisches Mischen der Komponenten (a), (b) und gegebenenfalls (c) und (d) mit dem Hilfsstoff durch gewöhnliche Methoden, wie z.B. durch einfaches Zusammenrühren der Einzelkomponenten erfolgen. Der UV-Absorber kann hierbei z.B. ohne weitere Behandlung, oder in mikronisiertem Zustand oder auch als Pulver eingesetzt werden.

Die kosmetischen Zusammensetzungen können beispielsweise Cremes, Gele, Lotionen, alkoholische und wässrig/alkoholische Lösungen, Sprays, Mischliposomen, Liposomengemische, Emulsionen, Wachs/Fett-Massen, Stiftpräparate, Puder oder Salben sein.

Als wasser- und ölhaltige Emulsionen (z.B. W/O-, O/W-, O/W/O-, W/O/W-Emulsionen oder-Mikroemulsionen) enthalten diese beispielsweise
0,1 bis 30 Gew.-%, vorzugsweise 0,1 bis 15 Gew.-% und insbesondere 0,5 bis 10 Gew.-%, bezogen auf das Gesamtgewicht der Zusammensetzung, der Komponente (a) und gegebenenfalls (b), (c) und (d),
1 bis 60 Gew.-%, insbesondere 5 bis 50 Gew.-% und vorzugsweise 10 bis 35 Gew.-%, bezogen auf das Gesamtgewicht der Zusammensetzung, mindestens einer Ölkomponente,
0 bis 30 Gew.-%, insbesondere 1 bis 30 Gew.-% und vorzugsweise 4 bis 20 Gew.-%, bezogen auf das Gesamtgewicht der Zusammensetzung, mindestens eines Emulgators,
10 bis 90 Gew.-%, insbesondere 30 bis 90 Gew.-%, bezogen auf das Gesamtgewicht der Zusammensetzung, an Wasser, und
0 bis 88,9 Gew.-%, insbesondere 1 bis 50 Gew.-%, weitere kosmetisch verträgliche Hilfsstoffe.

Als Ölkomponenten von ölhaltigen Zusammensetzungen (z.B. Öle, W/O-, O/W-, O/W/O-, W/O/W-Emulsionen oder-Mikroemulsionen) kommen beispielsweise Guerbetalkohole auf Basis von Fettalkoholen mit 6 bis 18, vorzugsweise 8 bis 10 Kohlenstoffatomen, Ester von linearen C₆-C₂₄-Fettsäuren mit linearen C₃-C₂₄-Alkoholen, Ester von verzweigten C6-C13-Carbonsäuren mit linearen C₆-C₂₄-Fettalkoholen, Ester von linearen C₆-C₂₄-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctylmalate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäu ren, Ester von C₆-C₂₄-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle (wie Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls), verzweigte primäre Alkohole, substituierte Cyclohexane, lineare und verzweigte C6-C22-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit insgesamt zwischen 12 bis 36 Kohlenstoffatomen, insbesondere 12 bis 24 C-Atomen, wie beispielsweise Di-n-octylether, Di-n-decylether, Di-n-nonylether, Din-undecylether, Di-n-dodecylether, n-Hexyl-n-octylether, n-Octyl-n-decylether, n-Decyl-nundecylether, n-Undecyl-n-dodecylether, n-Hexyl-n-undecylether, Di-tert-butylether, Di-isopentylether, Di-3-ethyldecylether, tert.-Butyl-n-octylether, iso-Pentyl-n-octylether und 2-Methyl-pentyl-n-octylether, Ringöffnungsprodukte von epoxidierten Fettsäureestern mit Polyolen, Siliconöle und/oder aliphatische bzw. naphthenische Kohlenwasserstoffe in Betracht. Von Bedeutung sind ferner Monoester der Fettsäuren mit Alkoholen mit 3 bis 24 C-Atomen. Bei dieser Stoffgruppe handelt es sich um die Produkte der Veresterung von Fettsäuren mit 8 bis 24 Kohlenstoffatomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckabspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise lsopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linoylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Von besonderer Bedeutung sind hierbei Isopropylmyristat, lsononansäure-C₁₆-C₁₈-Alkylester, Stearinsäure-2-ethylhexylester, Cetyloleat, Glycerintricaprylat, Kokosfettalkohol-caprinat/-caprylat und n-Butylstearat. Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglycol-dioleat, Ethylenglycol-di-isotridecanoat, Propylenglycol-di-(2-ethylhexanoat), Propylenglycol-di-isostearat, Propylenglycol-di-pelargonat, Butandiol-di-isostearat und Neopentylgycoldi-caprylat verwendbare Ölkomponenten dar.

Bevorzugte Mono- oder Polyole sind Ethanol, Isopropanol, Propylenglykol, Hexylenglycol, Glycerin und Sorbitol. Es können auch zwei- und/oder dreiwertige Metallsalze (Erdalkali, Al³⁺ u.a.) einer oder mehrerer Alkylcarbonsäuren verwendet werden.

Die Ölkomponenten können in einer Menge von beispielsweise 1 bis 60 Gew.-%, insbesondere 5 bis 50 Gew.-% und vorzugsweise 10 bis 35 Gew.-%, bezogen auf das Gesamtgewicht der Zusammensetzung, verwendet werden.

Für die Zusammensetzungen können jede konventionell einsetzbaren Emulgatoren verwendet werden.

Als Emulgatoren kommen beispielsweise nichtionogene Tenside aus den folgenden Gruppen in Betracht:
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und Alkylphenole mit 8 bis 15 C-Atomen in der Alkylgruppe, wie z.B. Ceteareth-20 oder Ceteareth-12;
- C12-C22-Fettsäuremono- und -diester von Anlagerungsprodukten von 1 bis 30 Mol Ethylenoxid an Polyole mit 3 bis 6 Kohlenstoffatomen, insbesondere an Glycerin;
- Glycerinmono- und -diester und Sorbitanmono- und -diester von gesättigten und ungesättigten Fettsäuren mit 6 bis 22 Kohlenstoffatomen und deren Ethylenoxidanlagerungsprodukte, wie z.B. Glyceryl Stearate, Glyceryl Isostearate, Glyceryl Oleate, Sorbitan Oleate oder Sorbitan Sesquioleate;
- C8-C22-Alkylmono- und -oligoglycoside und deren ethoxylierte Analoga, wobei Oligomerisierungsgrade von 1,1 bis 5, insbesondere 1,2 bis 1,4, und Glucose als Zuckerkomponente, bevorzugt sind;
- Anlagerungsprodukte von 2 bis 60 Mol, insbesondere 15 bis 60 Mol, Ethylenoxid an Ricinusöl und/oder gehärtetes Ricinusöl;
- Polyol- und insbesondere Polyglycerinester, wie z.B. Diisostearoyl Polyglyceryl-3 Diisostearate, Polyglyceryl-3 Diisostearate, Triglyceryl Diisostearate, Polyglyceryl-2 Sesquiisostearate oder Polyglyceryl Dimerate. Ebenfalls geeignet sind Gemische von Verbindungen aus mehreren dieser Substanzklassen;
- Partialester auf Basis linearer, verzweigter, ungesättigter bzw. gesättigter C6-C22-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose), wie z.B. Polyglyceryl-2-Dihydroxystearate oder Polyglyceryl-2-Diricinoleate;
- Mono-, Di- und Trialkylphosphate sowie Mono-, Di- und/oder Tri-PEG-alkylphosphate und deren Salze;
- Wollwachsalkohole;
- einer oder mehrere ethoxylierte Ester von natürlichen Derivaten, wie z.B. polyethoxylierte Ester von hydrogeniertem Castor-ÖI;
- Silikonöl-Emulgatoren wie z.B. Silikonpolyol;
- Polysiloxan-Polyalkyl-Polyether-Copolymere bzw. entsprechende Derivate, wie z.B. Cetyl Dimethicone Copolyol;
- Mischester aus Pentaerythrit, Fettsäuren, Citronensäure und Fettalkohol (vgl. DE-A-1165574) und/oder Mischester von Fettsäuren mit 6 bis 22 Kohlenstoffatomen, Methylglucose und Polyolen, vorzugsweise Glycerin oder Polyglycerin, wie z.B. Polyglyceryl-3 Glucose Distearate, Polyglyceryl-3 Glucose Dioleate, Methyl Glucose Dioleate oder Dicocoyl Pentaerythryl Distearyl Citrate sowie
- Polyalkylenglycole.

Die Anlagerungsprodukte von Ethylenoxid und/oder von Propylenoxid an Fettalkohle, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei in der Regel um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C₁₂-C₁₈-Fettsäuremono- und -diester von Anlagerungsprodukten von Ethylenoxid an Glycerin sind z.B. aus der DE-A-2024051 als Rückfettungsmittel für kosmetische Zubereitungen bekannt.

C8-C18-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden insbesondere solche oberflächenaktiven Verbindungen bezeichnet, die im Molekül mindestens eine quatäre Ammoniumgruppe und mindestens eine Carboxylat- und/oder Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat und 2-Alkyl-3-carboxymethyl-3-hydroxyethylimidazoline mit jeweils 8 bis 18 C-Atomen in der Alkyl- oder Acylgruppe sowie das Kokosacylaminoethylhyd roxyethylcarboxymethylg lycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden insbesondere solche verstanden, die ausser einer C₈-C₁₈-Alkyloder -Acylgruppe im Molekül mindestens eine freie Aminogruppe und mindestens eine -COOH oder -SO₃H-Gruppe enthalten und zur Ausbildung innerer Salze befähigt sind.

Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂-C₁₈-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Nichtionogene Emulgatoren sind bevorzugt. Unter den genannten nichtionogenen Emulgatoren sind ethoxylierte Fettalkohole mit 8 bis 22 Kohlenstoffatomen und 4 bis 30 EO-Einheiten besonders bevorzugt.

Die Emulgatoren können in einer Menge von beispielsweise 1 bis 30 Gew.-%, insbesondere 4 bis 20 Gew.-% und vorzugsweise 5 bis 10 Gew.-%, bezogen auf das Gesamtgewicht der Zusammensetzung, verwendet werden. Prinzipiell ist es aber auch möglich, auf die Verwendung von Emulgatoren zu verzichten.

Die erfindungsgemässen Zusammensetzungen, wie beispielsweise Cremes, Gele, Lotionen, alkoholische und wässrig/alkoholische Lösungen, Emulsionen, Wachs/Fett-Massen, Stiftpräparate, Puder oder Salben, können ferner als weitere Hilfs- und Zusatzstoffe milde Tenside, Überfettungsmittel, Perlglanzwachse, Konsistenzgeber, Verdickungsmittel, Polymere, Silikonverbindungen, Fette, Wachse, Stabilisatoren, biogene Wirkstoffe, Deowirkstoffe, Antischuppenmittel, Filmbildner, Quellbildner, weitere UV-Lichtschutzfaktoren, Antioxidantien, Hydrotrope, Konservierungsmittel, Insektenrepellentien, Solubilisatoren, Parfümöle, Farbstoffe, keimhemmende Mittel und dergleichen enthalten.

Als Überfettungsmittel können Substanzen wie beispielsweise Lanolin und Lecithin sowie polyethoxylierte oder acrylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als geeignete milde, d.h. besonders hautverträgliche Tenside sind z.B. Fettalkoholpolyglycolethersulfate, Monoglyceridsulfate, Mono- und/oder Dialkylsulfosuccinate, Fettsäureisethionate, Fettsäurearcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen, zu nennen.

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierten Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäure wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren und Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxymethylcellulose, ferner höhermolekulare Polyethylenglycolmono- und diester von Fettsäuren, Polyacrylate (z.B.

Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz oder Ammoniumchlorid.

Geeignete kationische Polymere sind beispielsweise kationische Cellulosederivate, wie z.B. eine quaternierte Hydroxymethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyllmidazol-Polymere, wie z.B. Luviquat® (BASF), Kondensationsprodukte von Polyglycolen und Aminen, quaternierte Kollagenpolypeptide, wie beispielsweise Lauryldimonium hydroxpropyl hydrolyzed collagen (Lamequat® L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der FR-A-2,252,840 sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinyacrylat-Copolymere, Vinylacetat/Butylmaleat/lsobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmetha Polyvinylacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm. Toil. 91, 27 (1976).

Typische Beispiele für Fette sind Glyceride, als Wachse kommen u.a. Bienenwachs, Carnaubawachs, Candelillawachs, Montanwachs, Paraffinwachs, hydrierte Ricinusöle, bei Raumtemperatur feste Fettsäureester oder Mikrowachse gegebenenfalls in Kombination mit hydrophilen Wachsen, z.B. Cetylstearylalkohol oder Partialglyceriden in Frage. Als Stabilisatoren können Metallsalze von Fettsäuren, wie z.B. Magnesium-, Aluminium- und/oder Zinkstearat bzw. -ricinoleat eingesetzt werden.

Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte, Nucleinsäuren/basen, phosphoylierte Basen und Oligonucleotide zu verstehen.

Als Deowirkstoffe kommen z.B. Antiperspirantien wie etwa Aluminiumchlorhydrate in Frage, Hierbei handelt es sich um farblose, hygroskopische Kristalle, die an der Luft leicht zerfliessen und beim Endampfen wässriger Aluminiumchloridlösungen anfallen. Aluminiumchlorhydrat wird zur Herstellung von schweisshemmenden und desodorierenden Zubereitungen eingesetzt und wirkt wahrscheinlich über den partiellen Verschluss der Schweissdrüsen durch Eiweiss- und/oder Polysaccharidfällung (vgl. J. Soc. Cosm. Chem. 24, 281 (1973)). Unter der Marke Locron® der Hoechst AG, Frankfurt(FRG, befindet sich beispielsweise ein Aluminiumchlorhydrat im Handel, das der Formel Al₂(OH)₅Cl x 2,5 H₂O entspricht und dessen Einsatz besonders bevorzugt ist (vgl. J. Pharm. Pharmacol. 26, 531 (1975)). Neben den Chlorhydraten können auch Aluminiumhydroxylacetate sowie saure Aluminium/Zirkoniumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, dass dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw. -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester. Antibakterielle Wirkstoffe, die die Keimflora beeinflussen und schweisszersetzende Bakterien abtöten bzw. in ihrem Wachstum hemmen, können ebenfalls in den Zubereitungen (insbesondere in den Stiftzubereitungen) enthalten sein. Beispiele hierfür sind Chitosan, Phenoxyethanol, Chlorhexidingluconat und 4(2-t-Butyl-5-methylphenoxy)phenol. Besonders wirkungsvoll hat sich auch 5-Chlor-2-(2,4-dichlorphenoxy)-phenol erwiesen( Irgasan® , Ciba Specialty Chemicals Inc.).

Zur Verbesserung des Fliessverhaltens können ferner Hydrotropiermittel, wie beispielsweise Ethanol, Isopropylalkohol oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind:
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methylolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffatomen im Alkylrest, wie beispielsweise Methyl- und Butylglucosid;
- Zuckeralkohole mit 5 bis 12 Kohlenstoffatomen, wie beispielsweise Sorbit oder Mannit;
- Zucker mit 5 bis 12 Kohlenstoffatomen, wie beispielsweise Gucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die in der Anlage 6, Teil A und B der Kosmetikverordnung aufgeführten weiteren Stoffklassen. Als Insekten-Repellentien kommen z.B. N,N-Diethyl-m-toluamid, 1,2-Pentandiol oder Insekten-Repellent 3535 in Frage.

Als Parfümöle seien genannt Gemische aus natürlichen und/oder synthetischen Riechstoffen. Natürliche Riechstoffe sind z.B. Extrakte von Büten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffe sind z.B. Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.Butylcyclohexylacetat, Linalylacetat, Dimethylbenzylcarbinylacetat, Phenylethylacetat, Linalybenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenwasserstoffatomen, Citral, Citronellal, Citronellyloxyacetaldehyd, Cyclamenaldehyd, Hydroxycitronellal, Lilial und Bourgeonal, zu den Ketonen z.B. die Jonone, u,u-lsomethylionon und Methylcedrylketon, zu den Alkoholen z.B. Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam ein ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, u-Damascone, Geraniumöl Bourbon, Cyclohexylsalicylat, Vertofix Coeur, iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, Seiten 81 bis 106 zusammengestellt sind. Die Farbstoffe werden üblicherweise in Konzentrationen von 0,001 bis 0,1 Gew.-%, bezogen auf die gesamte Mischung, eingesetzt.

Typische Beispiele für keimhemmende Mittel sind Konservierungsmittel mit spezifischer Wirkung gegen gram-positive Bakterien, wie etwa 2,4,4'-Trichlor-2'-hydroxydiphenylether, Chlorhexidin (1,6-Di-(4-chlorphenyl-biguanido)-hexan), Diclosan, 4-(2-t-butyl-5-methylphenoxy)phenol oder TCC (3,4,4'-Trichlorcarbanilid). Auch zahlreiche Riechstoffe und ätherische Öle weisen antimikrobielle Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Farnesol (3,7,11-Trimethyl-2,6,10-dodecatrien-1-ol), der im Lindenblütenöl vorhanden ist sowie Fraktionen aus der Wasserdampfextrakt des Holzes und/oder der Rinde von Callitris intratropica, welches auch als "blue cypress oil" bekannt ist. Auch Glycerinmonolaurat hat sich als Bakteriostatikum bewährt. Üblicherweise liegt der Anteil der zusätzlichen keimhemmenden Mittel bei 0,1 bis 2 Gew.-%, bezogen auf den Feststoffanteil der Zubereitungen.

Weiterhin können die kosmetischen Zusammensetzungen als Hilfsmittel Entschäumer, wie Silicone, Strukturanten, wie Maleinsäure, Lösungsvermittler, wie Ethylenglycol, Propylenglycol, Glycerin oder Diethylenglycol, Trübungsmittel, wie Latex, Styrol/PVP- oder Styrol/-Acrylamid-Copolymere, Komplexbildner, wie EDTA, NTA, u-Alanindiessigsäure oder Phosphonsäuren, Treibmittel, wie Propan-Butan-Gemische, N₂O Dimethylether, CO₂, N₂ oder Luft, sogenannte Kuppler- und Entwicklerkomponenten als Oxidationsfarbstoffvorprodukte, Reduktionsmittel, wie Thioglycolsäure und deren Derivate, Thiomilchsäure, Cysteamin, Thioäpfelsäure oder u-Mercaptoethansulfonsäure, oder Oxidationsmittel, wie Wasserstoffperoxid, Kaliumbromat oder Natriumbromat, enthalten.

Erfindungsgemässe kosmetische Formulierungen beinhalten die verschiedensten kosmetischen Mittel. Insbesondere kommen z.B. die folgenden Mittel in Betracht:
- Mittel zur Behandlung der Haut zu ihrer Aufhellung, Entfärbung oder Bleichung.
- Mittel zur Beseitigung von Sommersprossen, Alterflecken oder Melaninflecken oder Malen oder zur Behandlung von Überpigmentierung.
- Mittel zur Hautpflege, wie z.B. Hautwasch- und Reinigungsmittel in Form von stückförmigen oder flüssigen Seifen, Syndets oder Waschpasten,
- Hautpflegemittel, wie z.B. Körperlotions, Hautemulsionen, Mehrfachemulsionen oder Hautöle;
- Dekorative Körperpflegemittel, wie z.B. Gesichts-Make-ups in Form von Tages- oder Pudercremes, Gesichtspuder (lose und gepresst), Rouge oder Creme-Make-ups, Lippenpflegemittel, wie z.B. Lippenstift, Lip Gloss, Lippenkonturstift, Nagelpflegemittel, wie Nagellack, Nagellackentferner, Nagelhärter, oder Nagelhautentferner;
- Spezielle Deomittel und Antitranspirantien oder hornhautbeseitigende Mittel;
- Lichtschutzmittel, wie Sonnenmilche, -lotionen, -cremes, -öle, Sun-blockers oder Tropicals, oder After-sun-Präparate;
- Insektenabweisende Mittel ("Repellents"), wie z.B. Insektenöle, -lotionen, -sprays, oder -stifte;
- Deodorantien, wie Deosprays, Pumpsprays, Deogele, -stifte oder-roller;
- Antitranspirantien, wie z.B. Antitranspirantstifte, -cremes oder -roller;
- Mittel zur Reinigung und Pflege von unreiner Haut, wie z.B. Syndets (fest oder flüssig), Peeling- oder Scrubb-Präparate oder Peeling-Masken;
- Haarentfernungsmittel in chemischer Form (Depilation), wie z.B. Haarentfernungspulver, flüssige Enthaarungsmittel, cremige oder pastöse Enthaarungsmittel, Enthaarungsmittel in Gelform oder Aerosolschäume;
- Rasiermittel, wie z.B. Rasierseife, schäumende Rasiercremes, nichtschäumende Rasiercremes, -schäume, -gele, Preshave-Präparate für die Trockenrasur, Aftershaves oder Aftershave-Lotionen;
- Duftmittel, wie z.B. Duftwässer (Eau de Cologne, Eau de Toilette, Eau de Parfüm, Parfum de Toilette, Parfüm), Parfümöle oder Parfümcremes.

Diese aufgezählten Endformulierungen können in den verschiedensten Darreichungsformen vorliegen, wie z.B.
- in Form von flüssigen Zubereitungen als einer W/O- O/W-, O/W/O-, W/O/W-, PIT- und aller Arten von Mikroemulsionen
- in Form eines Gels,
- in Form eines Öls, einer Creme, Milch oder Lotion,
- in Form eines Make-Ups,
- in Form eines Stiftes,
- in Form eines Sprays (Spray mit Treibgas oder Pumpspray) oder eines Aerosols,
- in Form eines Schaumes, oder
- in Form einer Paste.

Von besonderer Bedeutung als kosmetische Zusammensetzungen für die Haut sind hierbei:
- Mittel zur Behandlung der Haut zu ihrer Aufhellung, Entfärbung oder Bleichung. zur Beseitigung von Sommersprossen, Alterflecken oder Melaninflecken oder Malen oder zur Behandlung von Überpigmentierung, wie Hautmilche, -lotionen, -cremes, -öle, -gele und Sprays sowie Pasten
- Lichtschutzmittel, wie Sonnenmilche, -lotionen, -cremes, -öle, Sun-blockers oder Tropicals, After-sun-Präparate. Von besonderem Interesse sind hierbei Sonnenschutzcremes, Sonnenschutzlotionen, Sonnenschutzöle, Sonnenschutzmilch sowie Sonnenschutzpräparate in Form eines Sprays.

Die folgenden Beispiele dienen zur Veranschaulichung der Erfindung, ohne sie auf diese zu beschränken.

### Beispiele

### Formulierungsbeispiele

### Beispiel 1: Grundformulierung

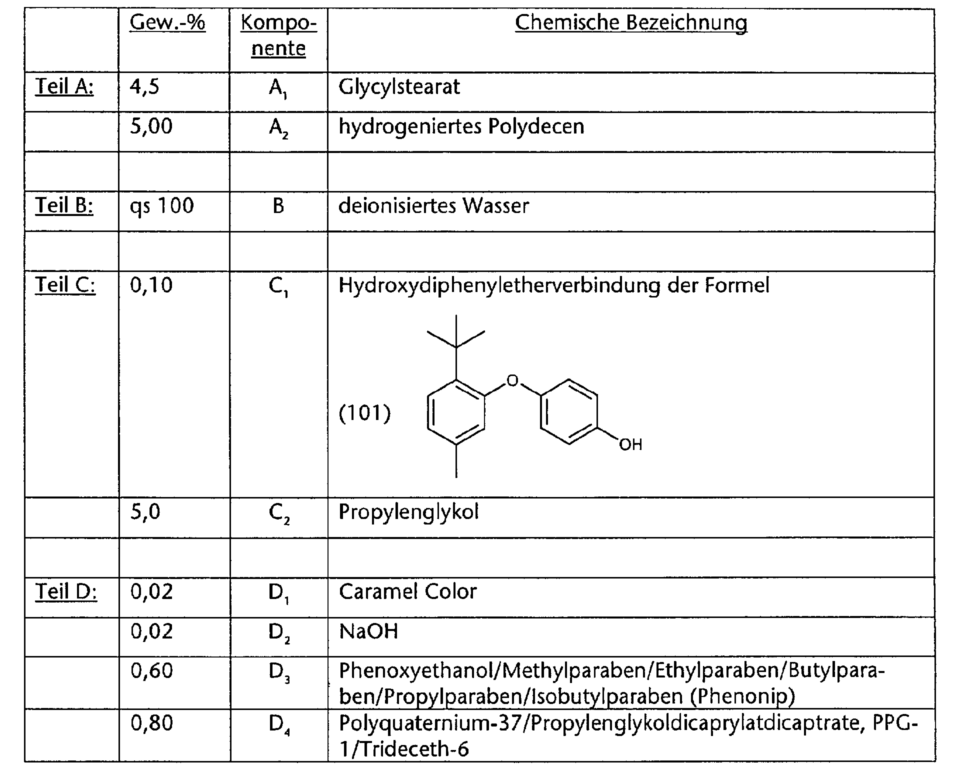

### Herstellung:

Teil A wird auf 75°C erhitzt. C₁ wird in C₂ dispergiert. Teil B wird auf 75°C erhitzt. Vor der Emulgierung gibt man Teil C zu Teil A. Teil A wird unter vorsichtigem Rühren zu Teil B gegeben. Anschliessend wird mit einem Ultra Turrax für 10 Sekunden homogenisiert.
Dann lässt man die Mischung unter vorsichtigem Rühren auf 60°C abkühlen und gibt D₂ in die Emulsion. D₃ wird unter Rühren hinzugegeben. Man rührt weiter, bis eine Temperatur von 40°C erreicht wird. Man gibt D₁ hinzu und stellt auf den pH-Wert von 6,7 ein.

### Beispiel 2: Formulierung Gesichtscreme

Eine Gesichtscreme wird hergestellt und enthält die folgenden Bestandteile:
3,0 Gew.-% Glycerinmonostearate,
1,5 Gew.-% Bienenwachs,
0,5 Gew.-% Sorbitanmonooleate,
5,0 Gew.-% Vaseline, liq.
10,0 Gew.-% Paraffin,
1,0 Gew.-% Lecithin,
0,5 Gew.-% Na N-Stearoyl-L-glutamat,
0,15 Gew.-% Xanthangum,
0,2 Gew.-% der Verbindung der Formel (101),
0,1 Gew.-% Kojic Säure,
0.1 Gew.-% Methylparaben, und
H₂O ad 100 Gew.-%.

### Beispiel 3: Formulierung Hautlotion

0,2 Gew.-% der Verbindung der Formel (101),
0,2 Gew.-% Kojisäure,
0,2 Gew.-% Polyoxyethylen,
3,0 Gew.-% der Verbindung der Formel (7e),
1,0 Gew.-% der Verbindung der Formel (10),
0.05 Gew.-% Castor Öl,
2 Gew.-% 1,3-Butylene glycol,
10 Gew.-% Ethanol,
Parfümöle, Stabilisatoren, und Wasser auf 100 Gewichtsanteile

### Beispiel 4: Formulierung eines Sprays

0,1 -0,5 Gew.-% der Verbindung der Formel (101)
0,5 - 0,1 Gew.-% Kojic Säure;
2,0 Gew.-% Solubilisant LRI (von Lcw),
70 Gew.-% Ethanol_{abs}
Wasser ad 100 %.

### Beispiel 5: Öl in Wasser Formulierung:

0,05-0,5 Gew.-% der Verbindung der Formel (101) und eine andere hautaufhellende Substanz;
0,05-5 Gew.-% eines oder mehrerer UV-Absorber;
12 Gew.-% Glycerylstearat;
6 Gew.-% Paraffin-Öl;
6 Gew.-% Caprylic/caprictriglycerid;
4 Gew.-% Glycerol;
0.2 Gew.-% Dinatrium EDTA
1.0 Gew.-% Citrat und
Wasser bis auf 100 Gewichtsanteile.

### Beispiel 6: O/W Lotion mit hautaufhellender Wirkung

| | INCl-Name | % w/w (äs supplied) |
|---|---|---|
| Teil A | Polyglyceryl-3 Methylglucose Distearate | 2.00 |
| | Mineral Oil | 5.70 |
| | Isopropyl Palmitate | 5.80 |
| | Caprylic/Capric Triglyceride | 6.50 |
| | Ethylhexyl Methoxycinnamate | 3.00 |
| | Cetyl Alcohol | 0.70 |
| Teil B | Glycerin | 3.00 |
| | Xanthan Gum | 0.25 |
| | Ethoxydiglycol | 5.00 |
| | Water | qs to 100 |
| Teil C | Phenoxyethanol (and) Methylparaben (and) Ethylparaben (and) Butylparaben (and) Propylparaben (and) Isobutylparaben | 0.70 |
| Teil D | Methylene Bis-Benzotriazolyl Tetramethylbutylphenol (and) Aqua (and) Decyl Glucoside (and) Propylene Glycol (and) Xanthan Gum | 10.00 |
| Part E | Water | 10.00 |
| | Alpha-Arbutin | 1.00 |
| Part F | Water (and) Citric Acid | qs to pH 4.5 - 5.0 |

### Technische Daten

| | |
|---|---|
| PH-Wert | 4.5 - 5.0 |
| UVA/UVB-Verhältnis* | 0.73 (berechnet) |
| Kritische Wellenlänge* | 384 nm (berechnet) |

| | |
|---|---|
| * gemessen auf einem Labsphere UV Transmittance Analyzer 2µl/cm² auf Transpore Tape | |

### Herstellung:

Teil A und B werden getrennt auf 75°C erhitzt. Teil A wird unter Rühren zu Teil B gegeben. Mit einem Ultra Turrax wird bei 10 000 Upm 30 sec homogenisiert. Man lässt auf 40°C abkühlen und inkorporiert Teil C in D (der pH-Wert von Tinosorb M wird vorher mit Zitronensäure t auf 5 eingestellt). Unter vorsichtigem Rühren wird weiter abgekühl und bei 30°C at 30°C Teil E inkorporiert. Der ph-Wert wird mit Teil F auf einen Wert zwischen 4.5 und 5.0 eingestellt.

### Beispiel 7: Hautaufhellende Lotion

| | INCl-Name | % w/w (as supplied) |
|---|---|---|
| Teil A | Polyglyceryl-3 Methylglucose Distearate | 2.20 |
| | Mineral Oil | 5.70 |
| | Isopropyl Palmitate | 5.80 |
| | Caprylic/Capric Triglyceride | 6.50 |
| | Ethylhexyl Methoxycinnamate | 3.00 |
| | Cetyl Alcohol | 0.80 |
| Teil B | Glycerin | 3.00 |
| | Hydroxyethylcellulose | 0.90 |
| | Water | qs to 100 |
| Teil C | Ethoxydiglycol | 5.00 |
| | Phenoxyethanol (and) Methylparaben (and) Ethylparaben (and) Butylparaben (and) Propylparaben (and) Isobutylparaben | 0.70 |
| Teil D | Methylene Bis-Benzotriazolyl Tetramethylbutylphenol (and) Aqua (and) Decyl Glucoside (and) Propylene Glycol (and) Xanthan Gum | 10.00 |
| Teil E | Water | 10.00 |
| | Alpha-Arbutin | 1.00 |
| Part F | Water (and) Citric Acid | qs to pH 4.5 - 5.0 |

### Technische Daten

| | |
|---|---|
| PH-Wert | 4.5-5.0 |
| UVA/UVB ratio* | 0.73 (berechnet) |
| Kritische Wellenlänge* | 384 nm (berechnet) |

| | |
|---|---|
| *gemessen mit einem Labsphere UV Transmittance Analyzer 2,µl/cm2 auf Transpore Tape | |

### Herstellung

Teil A und B werden getrennt auf 75°C erhitzt. Teil A wird Teil B unter Rühren hinzugegeben. Mit einem Ultra Turrax wird bei 10 000 Upm 30 sec. homogenisiert. Man lässt auf 40°C abkühlen und inkorporiert Teil C und D (der pH-Wert von Tinosorb M wird vorher mit Zitronensäure auf den Wert 5 eingestellt). Unter leichtem Rühren wird weiter abgekühlt und Teil E bei 30°C inkorporiert. Der ph-Wert wird mit Teil F auf einen Wert zwischen 4.5 und 5.0 eingestellt.

### Beispiel 8: Hautaufhellende Creme, Typ O/W

| | INCl-Name | % w/w (äs supplied) |
|---|---|---|
| Teil A | Cetearyl Alcohol (and) Dicetyl Phosphate (and) Ceteth-10 Phosphate | 4.00 |
| | C12-15 Alkyl Benzoate | 6.00 |
| | Cetearyl Ethylhexanoate (and) Isopropyl Myristate | 3.50 |
| | Caprylic/Capric Triglyceride | 2.00 |
| | Caprylyl Pyrrolidone | 1.50 |
| | Stearic Acid | 1.50 |
| | Glyceryl Stearate | 2.50 |
| | Bis-Ethylhexyloxyphenol Methoxyphenyl Triazine | 2.00 |
| Teil B | Water | qs to 100 |
| | Xanthan Gum | 0.1 |
| | Propylene Glycol | 3.50 |
| Teil C | Diazolidinyl Urea (and) lodopropynyl Butylcarbamate | 0.15 |
| Teil D | Ethoxydiglycol | 5.00 |
| Teil E | Water | 10.00 |
| | Alpha-Arbutin | 1.00 |
| Teil F | Water (and) Sodium Hydroxide | qs |
| | Fragrance | qs |
| PH-Wert: 4.5―5.0 | | |
| Viscosity (Brookfield DVIII+LV/rpm) 3500 - 5000 mPas | | |
| Kritische Wellenlänge* 371 nm | | |

| | | |
|---|---|---|
| * gemessen auf einem Labsphere UV Transmittance Analyzer 1.2 mg/cm² on PMMA support | | |

### Herstellung

Teil A und B werden getrennt auf 75°C erhitzt. Teil A wird unter zunehmendem Rühren zu Teil B gegeben. Danach wird mit einem Ultra Turrax bei 11 000 Upm 30 sec lang homogenisiert. Man lässt die Emulsion unter Rühren abkühlen. Bei 50°C werden Teil C und Teil D der Mischung hinzugegeben. Bei 30°C wird Teil E inkorporiert. Der pH-Wert wird mit NaOH-Lösung auf einen Wert zwischen 4.5 und 5.0 eingestellt. Zum Schluss wird das Parfüm hinzugegeben.

### Beispiel 9: O/W-Creme mit hautaufhellenden Eigenschaften

| | INCl-Name | % w/w (äs supplied) |
|---|---|---|
| Teil A | Cetearyl Alcohol (and) Dicetyl Phosphate (and) Ceteth-10 Phosphate | 4.00 |
| | C12-15 Alkyl Benzoate | 6.00 |
| | Cetearyl Ethylhexanoate (and) Isopropyl Myristate | 3.50 |
| | Caprylic/Capric Triglyceride | 2.00 |
| | Caprylyl Pyrrolidone | 1.00 |
| | Stearic Acid | 1.50 |
| | Glyceryl Stearate | 2.50 |
| | Ethylhexyl Methoxycinnamate | 3.00 |
| | Bis-Ethylhexyloxyphenol Methoxyphenyl Triazine | 1.50 |
| Teil B | Water | qs to 100 |
| | Xanthan Gum | 0.1 |
| | Propylene Glycol | 3.50 |
| Teil C | Diazolidinyl Urea (and) lodopropynyl Butylcarbamate | 0.15 |
| Teil D | Methylene Bis-Benzotriazolyl Tetramethylbutylphenol (and) Aqua (and) Decyl Glucoside (and) Propylene Glycol (and) Xanthan Gum | 5.00 |
| Part | Ethoxydiglycol | 5.00 |
| Part E | Water | 10.00 |
| | Alpha-Arbutin | 1.00 |
| Part F | Water (and) Sodium Hydroxide | qs |

### Technische Daten

| | |
|---|---|
| pH -Wert | 4.5-5.0 |

### Herstellung

Teil A und B werden getrennt auf 75°C erhitzt. Teil A wird unter zunehmendem Rühren zu Teil B gegeben. Danach wird mit einem Ultra Turrax bei 11 000 Upm 10 sec lang homogenisiert. Man lässt die Emulsion unter Rühren abkühlen. Bei 50°C werden Teil C und Teil D der Mischung hinzugegeben. Bei 30°C wird Teil E inkorporiert. Der pH-Wert wird auf einen Wert zwischen 4.5 und 5.0 eingestellt.

### Beispiel 11: Hautaufhellende O/W-Lotion

| | INCI-Name | % w/w (as supplied) |
|---|---|---|
| Teil A | Glyceryl Stearate (and) PEG-100 Stearate | 5.00 |
| | Stearyl Alcohol | 1.00 |
| | Tripalmitin | 0.70 |
| | Dimethicone | 2.00 |
| | C12-15 Alkyl Benzoate | 5.00 |
| | Isopropyl Palmitate | 5.00 |
| | Ethylhexyl Methoxycinnamate | 3.00 |
| Teil B | Water | qs to 100 |
| | Xanthan Gum | 0.25 |
| | Polysorbate 60 | 0.50 |
| | Glycerin | 3.00 |
| Teil C | Water | 10.00 |
| | Methylene Bis-Benzotriazolyl Tetramethylbutylphenol (and) Aqua (and) Decyl Glucoside (and) Propylene Glycol (and) Xanthan Gum | 8.00 |
| Teil D | Ethoxydiglycol | 5.00 |
| | Phenoxyethanol (and) Methylparaben (and) Ethylparaben (and) Butylparaben (and) Propylparaben (and) Isobutylparaben | 0.70 |
| Teil E | Water | 10.00 |
| | Alpha-Arbutin | 1.00 |
| Teil F | Water (and) Citric Acid | qs |

### Technische Daten

| | |
|---|---|
| PH-Wert | 4.50 - 5.00 |
| Aspekt | Weisse, leichte Lotion |

### Herstellung

Teil A und B werden getrennt auf 75°C erhitzt, Teil C auf 60°C. Danach wird Teil A unter Rühren zu Teil B gegeben. Danach wird mit einem Ultra Turrax bei 11 000 Upm 30 sec lang homogenisiert und Teil C inkorporiert. Man lässt auf 40°C abkühlen und gibt Teil D hinzu. Bei 30°C wird Teil E hinzugegeben. Bei Raumtemperatur wird der pH-Wert mit Teil F auf einen Wert zwischen 4.50 -5.00 eingestellt.

### Anwendungsbeispiele

### Beispiel 12:

An 10 asiatischen Testpersonen, bei denen beide Elternteile asiatischen Ursprungs sind, wird über mehrere Wochen an den Oberschenkeln (10 mg/cm²) die folgenden Testsubstanz aufgetragen:

| | |
|---|---|
| Formulierung 12a: (erfindungsgemäss) | 0,2 Gew.-% der Hydroxydiphenyletherverbindung der Formel (101) wird in 50% Ethanol gelöst und dann als Spray verwendet |

Der Aufhelleffekt wird mit einem Minolta Chromameter C-508-i (L*a*b-Farbraum) getestet.

Die Farbe der Hautflächen der Testpersonen wird
a. vor der Behandlung und
b. die Farbänderung als Unterschied zwischen L^{*}a^{*}b nach der Behandlung in Abständen von 4, 8 und 12 Wochen wie folgt bestimmt:

### Hautaufhellung L*:

Im L*a*b Farbsystem ist die Aufhellung definiert als L^{*}=0 (schwarz) und L*=100 (weiss).

### Rotton der Haut a*:

Im L*a*b Farbsystem ist der Rotton definiert als Zahl im Rot-Grün-Raum. Positive Zahlen zeigen eine Überwiegen von rot, 60 ist eine Farbe ohne rot. Negative Zahlen zeigen eine Überwiegen von grün, -60 ist eine Farbe ohne rot. je höher die Zahl, desto höher ist der Rotton der Haut.

### Gelbton der Haut b*

Im L*a*b Farbsystem ist der Gelbton definiert als die Zahl im Gelb-Blau-Raum. Positive Zahlen zeigen ein Überwiegen von gelb, 60 ist eine Farbe ohne blau. Negative Zahlen zeigen ein Überwiegen von blau, -60 ist eine Farbe ohne gelb. Je höher die Zahl, desto gelber ist die Haut.

**Tabelle 4:**

| | Behandlungsdauer (Wochen) | Form. 6a terfindungsgemäss) | Form. 6c (benchmark) | Form. 6d (benchmark) |
|---|---|---|---|---|
| Hautaufhellung L*: | 4 | 3.29 | 10.38 | 6.51 |
| | 8 | 8.5 | 13.04 | 10.88 |
| | 12 | 12.85 | 13.72 | 12.13 |
| Rotton a*: | 4 | -1.29 | -6.51 | -3.85 |
| | 8 | -3.58 | -8.85 | -8.93 |
| | 12 | -6.89 | -9.34 | -9.69 |
| Gelbton b | 4 | -2.45 | -3.88 | -1.18 |
| | 8 | -4.89 | -5.19 | -4.12 |
| | 12 | -7.29 | -5.81 | -3.76 |

Die Ergebnisse zeigen, dass nach einer Behandlungszeit von 12 Wochen eine bessere Wirkung der Aufhellung als beim Benchmark festgestellt werden kann.

### Beispiel 13: Hautaufhellende Seife

16 Probandinnen asiatischen Ursprungs (1. Grades, Vater und Mutter) seifen im doppelblinden, randomisierten Halbseitenvergleich12 Wochen lang 2x täglich nach dem Duschen eine vorgebebene Fläche auf den Unterarmen (ca. 5x 20 cm) und auf den Oberschenkeln (ca 10x25 cm) jeweils für 30 Sekunden mit Aktivseife oder mit Placeboseife ein. Dann werden die Seife durch Nachduschen entfernt.

Vor Beginn der Studie und nach 4 Wochen, 8 Wochen und 12 Wochen werden die Farbe der Prüfflächen mit einem Minolta 508i Spektralphotometer als L^{*}a^{*}b^{*} Werte bestimmt. Die entsprechenden Werte auf Oberschenkel und Unterarm werden linear gemittelt. Eine unbehandelte Fläche auf dem Oberarm dient jeweils als Kontrolle.

Veränderung der Helligkeit L* gegenüber entsprechenden Leerwerten.

| | | |
|---|---|---|
| Aktivseife | 4 Wochen | 1.92 (p=0.1, nicht signifikant); 3.38 (p<0.001); |
| | 12 Wochen | 5.22 (p<0.001). |
| Placebo | 4 Wochen | 1.15 (nicht signifikant); 0.65 (nicht signifikant); |
| | 12 Wochen | 1.10 (nicht signifikant). |

### Stückseifenformulierung

| INCl-Name | Aktivseife | Placebo |
|---|---|---|
| | % (as supplied) | % (as supplied |
| Hydroxydiphenyletherverbindung der Formel (101) | 0.5 | -- |
| Titandioxid | 0.2 | 0.2 |
| Tetra-Na-EDTA | 0.023 | 0.023 |
| Stearinsäure | 3.0 | 3.0 |
| Glycerin-Wasser | qs | qs |
| Vibracolor Green PGR7-1 | 0.004 | -- |
| Vibracolor Red PRE5-L | -- | 0.003 |
| Soap Noodles | ad 100 | ad 100 |

## Patentansprüche

1. Verwendung von
(a) Hydroxydiphenyletherverbindungen der Formel 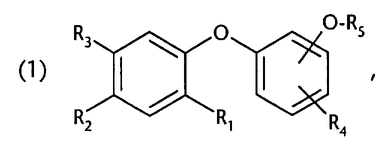 worin
R₁, R₂ und R₃ unabhängig voneinander Wasserstoff; Hydroxy; C₁-C₂₀-Alkyl; hydroxy-substituiertes C₁C₂₀-Alkyl; C₅-C₇-Cycloalkyl; C₁-C₂₀-Alkoxy; C₁-C₆-Alkylcarbonyl; Phenyl; oder Phenyl-C₁-C₃-Alkyl;
R₄ Wasserstoff, C₁-C₂₀ Alkyl; hydroxy-substituiertes C₁-C₂₀-Alkyl; C₅-C₇-Cycloalkyl; Hydroxy; Formyl; Acetonyl; Allyl; Carboxy; Carboxy-C₁-C₃-Alkyl; Carboxyallyl; C₂-C₂₀-Alkenyl; C₁-C₆-Alkylcarbonyl; C₁-C₃-Alkylcarbonyl-C₁-C3-Alkyl; Phenyl; oder Phenyl-C₁-C₃-Alkyl; und
R₅ Wasserstoff; C₁-C₂₀-Alkoxy; oder C₁-C₆-Alkylcarbonyl; bedeuten
als Melanogenese-Inhibitor und zum Aufhellen der Haut.

2. Verwendung nach Anspruch 1, **dadurch gekennzeichnet, dass** in Formel (1)
R₁R₂ und R₃ unabhängig voneinander Wasserstoff; C₁-C₂₀-Alkyl; hydroxy-substituiertes C₁-C₂₀-Alkyl; oder C₁-C₂₀-Alkoxy;
R₄ Wasserstoff; C₁-C₂₀-Alkyl; hydroxy-substituiertes C₁-C₂₀-Alkyl; Hydroxy; Formyl; Acetonyl; Allyl; Carboxymethyl; oder Carboxyallyl; und
R₅ Wasserstoff; C₁-C₂₀-Alkoxy; oder C₁-C₆-Alkylcarbonyl;
wobei mindestens einer der Substituenten R₁, R₂, R₃ und R₄ nicht Wasserstoff bedeutet bedeutet.

3. Verwendung nach Anspruch 1 oder 2, **dadurch gekennzeichnet, dass**
R₁ C₁-C₁₆-Alkyl; und
R₂, R₃ R₄und R₅ Wasserstoff;
bedeuten.

4. Verwendung nach Anspruch 1, **dadurch gekennzeichnet, dass** die Komponente (a) der Formel 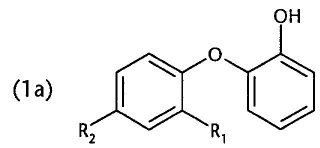 entspricht, worin
R₁ und R₂ unabhängig voneinander Wasserstoff; C₁-C₂₀-Alkyl; oder hydroxy-substituiertes C₁-C20-Alkyl; wobei mindestens einer der Substituenten R₁ und R₂ nicht Wasserstoff bedeutet, bedeuten.

5. Verwendung nach Anspruch 1, **dadurch gekennzeichnet, dass** die Komponente (a) der Formel 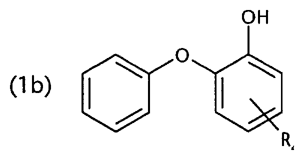 entspricht, worin
R₄ Carboxy; Carboxy-C₁-C₃-Alkyl; C₁-C₆-Alkylcarbonyl; oder C₁-C₃-Alkylcarbonyl-C₁-C₃-alkyl;
bedeutet.

6. Verwendung nach Anspruch 1, **dadurch gekennzeichnet, dass** die Komponente (a) der Formel 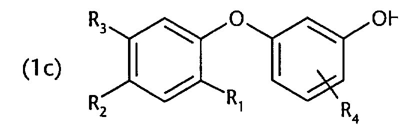 entspricht, worin
R₁, R₂ und R₃ unabhängig voneinander Wasserstoff; Hydroxy; C₁-C₂₀-Alkyl; oder hydroxy-substituiertes C₁C₂₀-Alkyl; und
R₄ C₁-C₂₀-Alkyl; hydroxysubstituiertes C₁-C₂₀-Alkyl; Phenyl; Phenyl-C₁-C₃-Alkyl; oder C₁-C₆-Alkylcarbonyl;
bedeuten.

7. Verwendung nach Anspruch 6, **dadurch gekennzeichnet, dass**
R₁,R₂ und R₃ Wasserstoff; oder C₁-C₂₀-Alkyl; bedeuten.

8. Verwendung nach Anspruch 6 oder 7, **dadurch gekennzeichnet, dass**
R₁,R₂ und R₃ Wasserstoff; und
R₄ C₁-C₂₀-Alkyl; Phenyl-C₁-C₅-Alkyl; oder C₁-C₆-Alkylcarbonyl;
bedeuten.

9. Verwendung nach Anspruch 1, **dadurch gekennzeichnet, dass** Komponente (a) der Formel 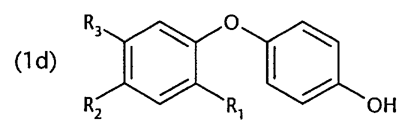 entspricht, worin
R₁, R₂ und R₃ unabhängig voneinander Wasserstoff; C₁-C₂₀-Alkyl; hydroxy-substituiertes C₁-C20-Alkyl; Cyclo-C₅-C₇-Alkyl; Phenyl-C₁-C₃-Alykl bedeuten, wobei mindestens einer der Substituenten R₁, R₂ oder R₃ nicht Wasserstoff bedeutet.

10. Verwendung nach Anspruch 9, **dadurch gekennzeichnet, dass**
R₁ und R₃ unabhängig voneinander C₁-C₂₀-Alkyl; und
R₂ Wasserstoff
bedeuten.

11. Verwendung nach einem der Ansprüche 1 to 10, **dadurch gekennzeichnet, dass** zusätzlich als Komponente (b) eine weitere hautaufhellende Substanz verwendet wird.

12. Verwendung nach einem der Ansprüche 1 bis 11, **dadurch gekennzeichnet, dass** die Komponente (b) ausgewählt ist aus Kojisäure, Arbutin, Quercitin, Aloesin, Azelainsäure, Guaiol, Ellagsäure und ihren Esterverbindungen.

13. Verwendung nach einem der Ansprüche 1 bis 12, **dadurch gekennzeichnet, dass** das Verhältnis der Komponente (a) : (b) 1 : 99, vorzugsweise 5 :95, und insbesondere 10 : 90 bis 99 : 1, vorzugsweise 95 : 5, und insbesondere 90 : 10 Gew.-% der Komponente (b) ist.

14. Verwendung nach einem der Ansprüche 1 bis 13, **dadurch gekennzeichnet, dass** die Zusammensetzungen zusätzlich als Komponente (c) einen oder mehrere UV-A- und/oder UV-B Absorber enthalten.

15. Verwendung nach Anspruch 14, **dadurch gekennzeichnet, dass** als UV-A- bzw. UV-B Absorber eine Verbindung der Formel 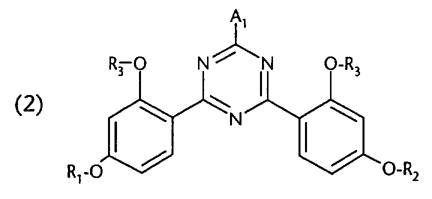 verwendet wird, worin
R₁ und R₂, unabhängig voneinander, C₁-C₁₈-Alkyl; C₂-C₁₈-Alkenyl; einen Rest der Formel -CH₂-CH(-OH)-CH₂-O-T₁ ; oder
R₁ und R₂ einen Rest der Formel 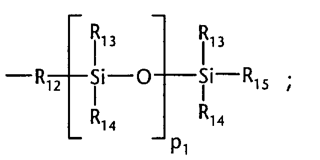
R₁₂ die direkte Bindung; einen geradkettigen oder verzweigten C₁-C₄-Alkylenrest oder einen Rest der Formel ―Cₘ₁ H_{2m1―} oder ―Cₘ₁H₂ₘ₁―O―;
R₁₃, R₁₄ und R₁₅, unabhängig voneinander, C₁ -C₁₈-Alkyl; C₁-C₁₈-Alkoxy oder einen Rest der Formel 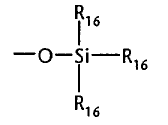
R₁₆ C₁-C₅-Alkyl;
m₁ und m₃, unabhängig voneinander, 1 bis 4;
p₁ 0 oder eine Zahl von 1 bis 5;
A₁ einen Rest der Formel 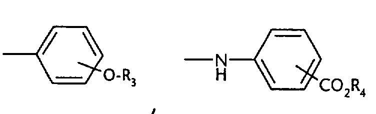 oder der Formel 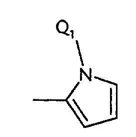
R₃ Wasserstoff; C₁-C₁₀-Alkyl, -(CH₂CHR₅-O)_{n₁}-R₄ ; oder einen Rest der Formel -CH₂-CH(-OH)-CH₂-O-T₁;
R₄ Wasserstoff; M; C₁-C₅-Alkyl; oder einen Rest der Formel (CH₂)_{m₂}-O-T₁;
R_{5,} Wasserstoff; oder Methyl;
T₁ Wasserstoff; oder C₁-C₈-Alkyl;
Q₁ C₁-C₁₈-Alkyl;
M ein Metallkation;
m₂ 1 bis 4; und
n₁ 1-16;
bedeuten.

16. Verwendung nach Anspruch 14 oder 15, **dadurch gekennzeichnet, dass** die Zusammensetzung als Komponente (c) die Verbindung der Formel 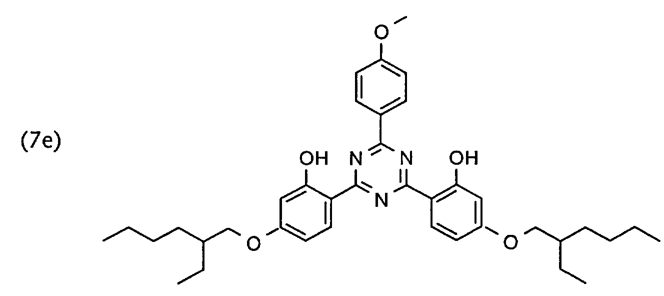 enthält.

17. Verwendung nach Anspruch 14 oder 15, **dadurch gekennzeichnet, dass** die Zusammensetzung als Komponente (c) die Verbindung der Formel 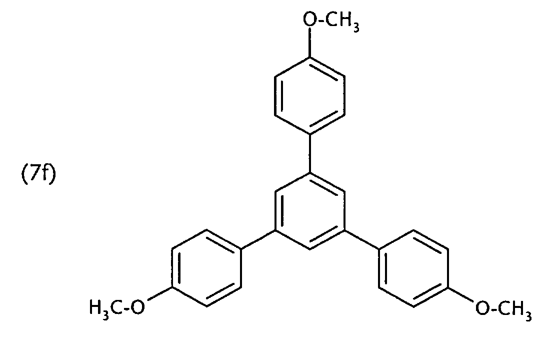 enthält.

18. Verwendung Anspruch 14, **dadurch gekennzeichnet, dass** die Zusammensetzung als Komponente (c) die Verbindung der Formel 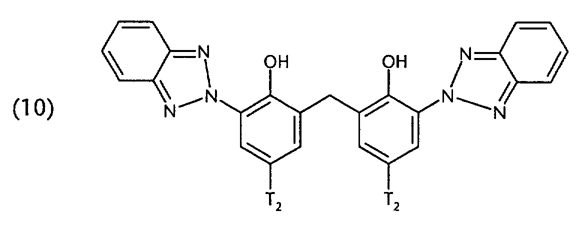 enthält, worin
T₂ C₁-C₁₂-Alkyl bedeutet.

19. Verwendung nach Anspruch 186, **dadurch gekennzeichnet, dass**
T₂ iso-Octyl bedeutet.

20. Verwendung nach Anspruch 14, **dadurch gekennzeichnet, dass** die Zusammensetzung als Komponente (c) Octylmethoxycinnamat enthält.

21. Verwendung nach Anspruch 14, **dadurch gekennzeichnet, dass** die Zusammensetzung als Komponente (c) Benzophenon 3 enthält.

22. Verwendung von Zusammensetzungen nach einem der Ansprüche 1 bis 21 zur Aufhellung der Haut.

23. Verwendung der Zusammensetzungen nach einem der Ansprüche 1 bis 21 in kosmetischen Formulierungen.

24. Verwendung nach Anspruch 23, **dadurch gekennzeichnet, dass** sie als Öl in Wasser Formulierungen, in Lösungsmittel-Formulierungen, oder als Pasten-Formulierungen verwendet werden.

25. Verwendung nach Anspruch 23 oder 24, **dadurch gekennzeichnet, dass** der Anteil der Mischung in der Formulierung zwischen 0,01% und 10% Gewichtsanteilen liegt.

26. Kosmetische Formulierung, enthaltend
a) eine Verbindung der Formel (1); und gegebenenfalls
(b) weitere die Haut aufhellenden Wirksubstanzen, und gegebenenfalls
(c) einen oder mehrere UV-A- und/oder UV-B Absorber, und gegebenenfalls
(d) ein Antioxidans sowie kosmetisch verträgliche Hilfs- oder Trägerstoffe enthält.

27. Kosmetische Formulierung nach Anspruch 26, **dadurch gekennzeichnet, dass** sie 0.001 bis 10, vorzugsweise 0,05 bis 1 Gew.-% der Komponente (a),
0 bis 10, vorzugsweise 0,05 bis 1 Gew.-% der Komponente (b),
0 bis 30 Gew.-%, vorzugsweise 0,1 bis 15 Gew.-% der Komponente (c) und
0 bis 30 Gew.-%, vorzugsweise 0,1 bis 15 Gew.-% der Komponente (d)
enthält.
